(19) **Europäisches Patentamt / European Patent Office / Office européen des brevets**

(11) **EP 2 855 664 B1**

(12) **EUROPEAN PATENT SPECIFICATION**

(45) Date of publication and mention of the grant of the patent:
**04.10.2017 Bulletin 2017/40**

(51) Int Cl.:
*C12N 5/073* (2010.01)     *G01N 33/50* (2006.01)
*G06K 9/00* (2006.01)     *G06K 9/62* (2006.01)

(21) Application number: **13729621.6**

(22) Date of filing: **31.05.2013**

(86) International application number:
**PCT/EP2013/061260**

(87) International publication number:
**WO 2013/178785 (05.12.2013 Gazette 2013/49)**

(54) **EMBRYO QUALITY ASSESSMENT BASED ON BLASTOCYST DEVELOPMENT**

EMBRYO-QUALITÄTSBEURTEILUNG BASIEREND AUF DER BLASTOZYSTENENTWICKLUNG

ÉVALUATION DE LA QUALITÉ D'UN EMBRYON SUR LA BASE DU DÉVELOPPEMENT DU BLASTOCYSTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **31.05.2012 PCT/DK2012/050188
25.06.2012 US 201261663856 P
29.06.2012 EP 12174432
28.09.2012 US 201261707321 P**

(43) Date of publication of application:
**08.04.2015 Bulletin 2015/15**

(73) Proprietor: **Unisense FertiliTech A/S
8200 Aarhus N (DK)**

(72) Inventors:
• **RAMSING, Niels B
  DK-8240 Risskov (DK)**
• **KRISTENSEN, Morten
  DK-9500 Hobro (DK)**
• **LÆGDSMAND, Mette
  DK-8830 Tjele (DK)**
• **KUHLMAN, Reidun Berghold
  DK-8420 Knebel (DK)**
• **AGERHOLM, Inge Errebo
  DK-8740 Brædstrup (DK)**
• **ISAKSEN, Mai Faurschou
  DK-8270 Højbjerg (DK)**
• **GUNDERSEN, Jens K
  DK-8260 Viby J. (DK)**

(74) Representative: **D Young & Co LLP
120 Holborn
London EC1N 2DY (GB)**

(56) References cited:
**WO-A1-2011/025736**

• **CRUZ M ET AL: "Time-lapse video analysis provides a correlation between early embryo division kinetics and subsequent blastocyst formation and quality", HUMAN REPRODUCTION (OXFORD), vol. 26, no. Suppl. 1, July 2011 (2011-07) , page I167, XP002700044, & 27TH ANNUAL MEETING OF THE EUROPEAN-SOCIETY-OF-HUMAN-REPRODUCTION-AND -EMBRYOLOGY; STOCKHOLM, SWEDEN; JULY 03 -06, 2011**
• **K. KIRKEGAARD ET AL: "Human embryonic development after blastomere removal: a time-lapse analysis", HUMAN REPRODUCTION, vol. 27, no. 1, 1 January 2012 (2012-01-01), pages 97-105, XP055069559, ISSN: 0268-1161, DOI: 10.1093/humrep/der382**
• **M. MESEGUER ET AL: "The use of morphokinetics as a predictor of embryo implantation", HUMAN REPRODUCTION, vol. 26, no. 10, 9 August 2011 (2011-08-09), pages 2658-2671, XP055033123, ISSN: 0268-1161, DOI: 10.1093/humrep/der256**

- **LEMMEN J G ET AL: "Kinetic markers of human embryo quality using time-lapse recordings of IVF/ICSI-fertilized oocytes", REPRODUCTIVE BIOMEDICINE ONLINE, REPRODUCTIVE HEALTHCARE LTD, GB, vol. 17, no. 3, 1 January 2008 (2008-01-01), pages 385-391, XP003028429, ISSN: 1472-6483 [retrieved on 2008-07-30]**

## Description

The present invention relates to a method and to a system for selecting embryos for in vitro fertilization based on observed cell kinetics and cell morphology, in particular the embryo development in the later stages from the initiation of compaction and to the formation of the blastocyst.

## Background of invention

Infertility affects more than 80 million people worldwide. It is estimated that 10% of all couples experience primary or secondary infertility. In vitro fertilization (IVF) is an elective medical treatment that may provide a couple who has been otherwise unable to conceive a chance to establish a pregnancy. It is a process in which eggs (oocytes) are taken from a woman's ovaries and then fertilized with sperm in the laboratory. The embryos created in this process are then placed into the uterus for potential implantation. To avoid multiple pregnancies and multiple births, only a few embryos are transferred (normally less than four and ideally only one). Selecting proper embryos for transfer is a critical step in any IVF-treatment. Current selection procedures are mostly entirely based on morphological evaluation of the embryo at different timepoints during development and particularly an evaluation at the time of transfer using a standard stereomicroscope. However, it is widely recognized that the evaluation procedure needs qualitative as well as quantitative improvements.

One approach is to use 'early cleavage' to the 2-cell stage, (i.e. before 25 - 27 h post insemination/injection), as a quality indicator. In this approach the embryos are visually inspected 25 - 27 hours after fertilization to determine if the first cell cleavage has been completed. However, although the early cleavage as well as other early criteria may be a quality indicator for development into an embryo there is still a need for quality indicators for implantation success and thereby success for having a baby as a result. Kirkegaard et al in " Human embryonic development after blastomere removal: a time-lapse analysis", Human Reproduction, Published for the European Society of Human Reproduction and Embryology by IRL Press, Vol:27, Nr:1, Pages:97-105 discloses a study of embryos from couples undergoing preimplantation genetic diagnosis to evaluate the effect of blastomere biopsy on early human embryonic development using time-lapse analysis.

Cruz et al in "Time-lapse video analysis provides a correlation between early embryo division kinetics and subsequent blastocyst formation and quality", Human Reproduction; 27th annual meeting of the European Society of Human Reproduction and Embryology; Stockholm, Sweden; July 3-6, 2011, Oxford Journals, Vol:26, Nr:Suppl. 1, Page I167 discloses a method of time-lapse video analysis providing a correlation between early embryo division kinetics and subsequent blastocyst formation and quality.

M. Meseguer et al, "The Use of Morphokinetics as a Predictor of Embryo Implantation", Human Reproduction, no. 10, ISSN 0268-1161, page 2658 - 2671 discloses an IVF incubator with a built-in camera designed to automatically acquire images at defined time points to simultaneously monitor 72 embryos without removing the embryos from the controlled environment. Analysis of cleavage times, blastomere size and multinucleation was made for 247 transferred embryos with either failed or full implantation. Several parameters were found to be correlated with subsequent implantation (e.g. time of first and subsequent cleavages as well as the time between cleavages).

WO2011025736 discloses methods, compositions and kits for determining the developmental potential of one or more embryos or pluripotent cells and/or the presence of chromosomal abnormalities in one or more embryos or pluripotent cells are provided. These methods, compositions and kits find use in identifying embryos and oocytes in vitro that are most useful in treating infertility in humans.

Lemmen J G et al in "Kinetic markers of human embryo quality using time-lapse recordings of IVF/ICSI-fertilized oocytes", Reproductive Biomedicine Online, Reproductive Healthcare Ltd, GB, vol. 17, no. 3, pages 385 - 391 discloses a time-lapse system used to study the timing and coordination of events during early development from zygote to cleavage stage embryo. The aim was to identify markers linked to good-quality embryos and implantation. Early disappearance of pronuclei and onset of first cleavage after fertilization was correlated with a higher number of blastomeres on day 2 after oocyte retrieval. In addition, synchrony in appearance of nuclei after the first cleavage was significantly associated with pregnancy success (P < 0.05).

## Summary of invention

Aspects and features of the present invention are defined in the appended claims.

A recent study (Wong et al. (2010)) focused on the embryo development before embryonic genome activation (EGA) indicating that success and failure in human embryo development is largely determined before EGA, thereby justifying an early implantation at day 2 after fertilization. A pending application from the present applicant PCT/DK2012/05018 ("Embryo quality assessment based on blastomere cleavage and morphology") filed 31.05.2012 monitors the timing and duration of the subsequent cleavages, wherein embryonic genome activation takes place, and that led to additional embryo quality criteria. However, the present inventors have found that monitoring the embryo all the way to the blastocyst stages, during which the embryonic genome activation has taken over completely, may lead to a range of blastocyst quality criteria that are very useful in the selection of embryos that has transformed into blastocysts, in order to increase implantation success. Traditionally blastocysts have been evaluated based on the number of cells in the trophectoderm or in the inner cell mass, but these blastocyst parameters are difficult to quantify. A purpose of the present invention is therefore to develop new blastocyst quality criteria that are easier to quantify in order to evaluate embryo quality at the blastocyst stage.

Accordingly, the present invention relates to a method and to a system to facilitate the selection of optimal in vitro fertilized embryos to be transferred for implantation after their transformation into blastocysts based on morphological and/or kinetic parameters extracted during their development. The blastocyst quality criteria may advantageously be combined with earlier embryo quality parameters, e.g. as listed in WO 2007/144001 and in pending PCT application PCT/DK2012/05018 entitled "Embryo quality assessment based on blastomere cleavage and morphology" filed at 31.05.2012 and thereby additional information of embryo quality and embryo viability.

**Definitions and embryo quality parameters**

Cleavage time is defined as the first observed timepoint when the newly formed blastomeres are completely separated by confluent cell membranes. The cleavage time is therefore the time of completion of a blastomere cleavage. In the present context the times are expressed as hours post ICSI microinjection or post time for mixing of semen and oocyte in IVF, i.e. the time of insemination. This is the time of the deliberate introduction of sperm into the ovum. However, herein the term fertilization is also used to describe this timepoint. Thereby the cleavage times are as follows:

- $t2$: Time of cleavage to 2 blastomere embryo
- $t3$: Time of cleavage to 3 blastomere embryo
- $t4$: Time of cleavage to 4 blastomere embryo
- $t5$: Time of cleavage to 5 blastomere embryo
- $t6$: Time of cleavage to 6 blastomere embryo
- $t7$: Time of cleavage to 7 blastomere embryo
- $t8$: Time of cleavage to 8 blastomere embryo

Duration of cell cycles is defined as follows:

- $cc1 = t2$: First cell cycle.
- $cc2 = t3-t2$: Second cell cycle, duration of period as 2 blastomere embryo.
- $cc2b = t4-t2$: Second cell cycle for both blastomeres, duration of period as 2 and 3 blastomere embryo.
- $cc3 = t5-t3$: Third cell cycle, duration of period as 3 and 4 blastomere embryo.
- $cc2\_3 = t5-t2$: Second and third cell cycle, duration of period as 2, 3 and 4 blastomere embryo.
- $cc4 = t9-t5$: Fourth cell cycle, duration of period as 5, 6, 7 and 8 blastomere embryo.

Synchronicities are defined as follows:

- $s2 = t4-t3$: Synchrony in division from 2 blastomere embryo to 4 blastomere embryo.
- $s3 = t8-t5$: Synchrony in division from 4 blastomere embryo to 8 blastomere embryo.
- $s3a = t6-t5$; $s3b = t7-t6$; $s3c = t8-t7$: Duration of the individual cell divisions involved in the development from 4 blastomere embryo to 8 blastomere embryo.

Cleavage period: The period of time from the first observation of indentations in the cell membrane (indicating onset of cytoplasmic cleavage) to the cytoplasmic cell cleavage is complete so that the blastomeres are completely separated by confluent cell membranes. Also termed as duration of cytokinesis.

Fertilization and cleavage are the primary morphological events of an embryo, at least until the 8 blastomere stage. Cleavage time, cell cycle, synchrony of division and cleavage period are examples of morphological embryo parameters that can be defined from these primary morphological events and each of these morphological embryo parameters are defined as the duration of a time period between two morphological events, e.g. measured in hours.

A normalized morphological embryo parameter is defined as the ratio of two morphological embryo parameters, e.g. $cc2$ divided by $cc3$ ($cc2/cc3$), or $cc2/cc2\_3$ or $cc3/t5$ or $s2/cc2$.

The duration of a plurality of cell cycles (e.g. CC1, CC2, CC3 and CC4) can be combined to form a common normalized parameter:

$$CC_{norm} = \sqrt{\sum_{all\ i} \left(\frac{CCi - CCi_{median}}{CCi_{median}}\right)^2}$$

where CCi e.g. is selected from CC1 to CC4. In one embodiment of the invention a high value of $CC_{norm}$ indicates a poor embryo quality as one or more of the variables CCi is far from the median, i.e. it is not the absolute values of CCi that are used, but the mutual relation of the variables. The median may be calculated based on the whole population or parts of the population (e.g. embryos with known and positive implantation). Another equivalent variable using the logarithmic value instead ($ICC_{norm}$) may also be useful in assessing embryo quality.

$$lCC_{norm} = \sqrt{\sum_{all\ i} w_i log \left(\frac{CCi - CCi_{median}}{CCi_{median}}\right)^2}$$

Likewise the synchronicity Si of the cell divisions (e.g. S2, S3 and S4) may be combined to form a common normalized parameter:

$$S_{norm} = \sqrt{\sum_{all\ i} \left(\frac{Si}{Si_{median}}\right)^2}$$

In one embodiment of the invention a high value of $S_{norm}$ indicates a poor embryo quality as one or more of the synchronicities is long compared to the. Another equivalent variable using the logarithmic value instead ($IS_{norm}$) may also be useful in assessing embryo quality.

$$lS_{norm} = \sqrt{\sum_{all\ i} w_i log \left(\frac{Si}{Si_{median}}\right)^2}$$

The variables $CC_{norm}$ and $S_{norm}$ may be calculated based on the first, second, third or fourth cell cycle, depending on the duration of the incubation.

The pending application PCT/DK2012/050236 filed 29 June 2012 and entitled "Adaptive embryo selection criteria optimized through iterative customization and collaboration" from the same applicant relates to the issue of adapting embryo quality criteria across populations of embryos cultures under different incubation conditions, e.g. in different clinics. This application is hereby incorporated by reference in its entirety. However, quality parameters like $CC_{norm}$, $ICC_{norm}$, $S_{norm}$ and $IS_{norm}$ may help to ensure that quality models will be directly applicable across different populations of embryos cultured under different incubation conditions, because they are based on variables that are insensitive to differences in running conditions. Another example of that is quality parameters based on relative time periods (e.g. CC2/CC3), variables divided with a central estimate of that variable (e.g. mean or median, e.g. cc2/cc2_median) or using target intervals where the center is scaled according to a central estimate and the boundaries are scaled according to a variance estimate (e.g. variance, standard deviation, percentiles).

The following discrete (binary) variables can be used

- MN2: Multi nucleation observed at the 2 blastomere stage; can take the values "True" or False".
- MN2val: the number of multinuclear cells at the 2 cell stage (0,1,2).
- MN4: Multi nucleation observed at the 4 blastomere stage; can take the values "True" or False".
- MN4val: the number of multinuclear cells at the 4 cell stage (0,1,2,3,4).
- EV2: Evenness of the blastomeres in the 2 blastomere embryo; can take the values "True" (i.e. even) or "False" (i.e. uneven).

*Blastocyst related parameters*

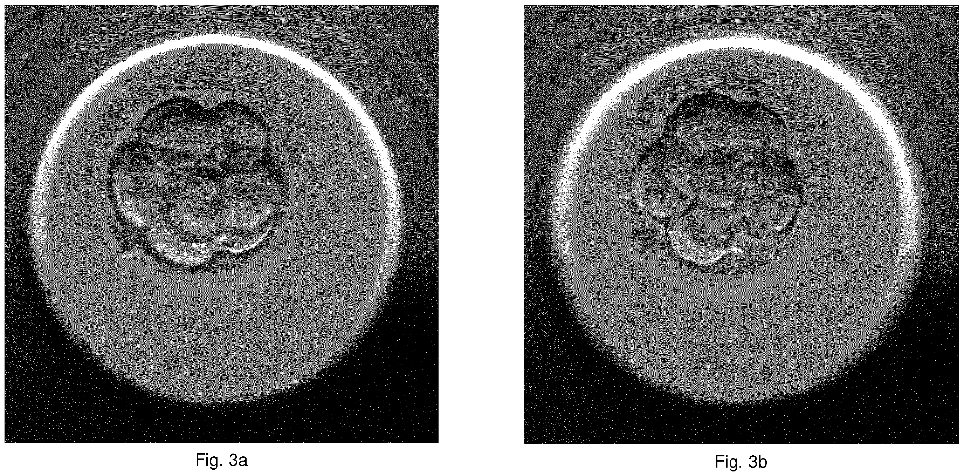

A blastocyst quality criterion is an example of an embryo quality criterion. The blastocyst quality criteria relate to the development of the embryo from compaction, i.e. initial compaction, to the hatched blastocyst. Compaction is a process wherein an intensification of the contacts between the blastomeres with tight junction and desmosomes result in reduction of the intercellular space and a blurring of the cell contours (see fig. 3). Before compaction the blastomeres of the embryo can be followed individually and before compaction the embryo development follow a route of distinct and mostly synchronous cell divisions that can be observed by the naked eye and easily annotated. After compaction the embryo development is characterized by a more or less continuous development from morula to blastocyst, where individual blastomeres are very difficult to track, but a number of stages are visually characteristic and can be annotated to become blastocyst related parameters. The following blastocyst related parameters may be used:

Initial compaction (IC) describes the first time a compaction between 2 or more blastomeres is observed. Thus, IC marks the initiation of the compaction process.

Morula (M) is defined as the first time where no plasma-membranes between any blastomeres are visible. When the compaction process is complete no plasma-membranes between any of the blastomeres forming the compaction are visible and the embryo can be defined as a morula. Most often Morula is seen after S3 close to or right in the beginning of the fourth synchrony period (S4). Rarely do the embryos cleave to 16 cell or more before compaction is initiated.

Initial differentiation of trophectoderm (IDT) is defined as the first time during the morula stage where distinct trophectoderm cells are recognized. It describes the onset of differentiation of the trophectoderm cells. The blastomeres gradually become flattened and elongate creating a barrier between the outside environment and the inner cell part of the morula.

Early blastocyst (ERB) is defined as the first time a fluid-filled cavity, the blastocoel, can be observed. It is also referred to as "Onset of cavitation". It describes the initiation of the transition period between the morula stage and the blastocyst stage of the embryo. Embryos often remain in this transition stage for a period of time before entering the actual blastocyst stage. The onset of cavitation usually appears immediately after differentiation of the trophectoderm cells. The outer layer of the morula with contact to the outside environment begins to actively pump salt and water into the intercellular space, as a result of which a cavity (the blastocoel) begins to form.

Blastocyst (Bl) is defined as where the fluid filled cavity is finally formed, i.e. the cavity does not increase significantly anymore before the blastocyst starts to expand (tEB)

Initial differentiation of inner cell mass (IDCIM) defined as the first time the inner cell mass can be recognized. IDCIM describes the initiation of inner cell mass development. An eccentrically placed cluster of cell connected of gab junction where the boundaries between the cells seem not well defined.

Onset of expansion of the blastocyst (EB) is defined as the first time the embryo has filled out the periviteline space and starts moving/expanding Zona Pelucidae. EB describes the initiation of the embryos expansion. As the blastocyst expands the zona pellucida becomes visibly thinner.

Hatching blastocyst (HB) is defined as the first time a trophectoderm cell has escaped / penetrated the zona pellucida.

Fully hatched blastocyst (FH) is defined as when hatching is completed with shedding zona pellucida.

Number of Contractions (NC (X)) describes the number of contractions (X) the embryo undergoes after the onset of cavitation. In many embryos the contractions can be quite large and lead to a large reduction of the embryonic volume. A contraction is defined as a reduction in the cross sectional surface area of the embryo of more than 15%.

Degree of vacuolization (VC (X); X={0, 1, 2, 3}) describes the extent of vacuolization after initiation of the morula stage. The degree of the vacuoles is rated by a 0-3 scale (0 = no vacuolization; 1 = small degree of vacuolization where small vacuoles appear but the embryonic development does not appear to be affected; 2 = moderate degree of vacuolization where large vacuoles appear and embryonic development is affected to some extent; 3 = severe vacuolization where very large vacuoles appear and embryonic development is severely affected. In this incidence the vacuoles can be mistaken for the blastocyst cavitation.

Partial Compaction (PC) describes an uneven compaction where one or more of the blastomeres are not included in the compaction process.

In humans embryonic gene activation (EGA) typically occurs on day 3, around the 8-cell stage. Before EGA embryos are observed to translate only maternally inherited mRNA, i.e. that mRNA which is present in the oocyte when it is fertilized. The mRNA is localized in different parts of the oocyte, so that as the oozyte/zygote divides it is segregated into different blastomeres. This segregation is thought to underlie much of the differentiation of cells that occurs before EGA. After EGA the embryo begins to transcribe its own DNA, cells become motile and cell division becomes asynchronous. Since the cells are now transcribing their own DNA, this stage is where differential expression of paternal genes is first observed. The transition around EGA is also referred to as midblastula or midblastula transition.

*Chromosomal content*

Aneuploidy is an abnormal number of chromosomes and is a type of chromosome abnormality. Aneuploid embryos can have one or more missing chromosomes and/or one or more extra chromosomes. Aneuploidy occurs during cell division when the chromosomes do not separate properly between the two cells. An aneuploid embryo is an embryo which contains an aneuploidy. Correspondingly a euploid embryo is an embryo that is characterized as being chromosomally normal. Euploid (i.e. normal) embryos have the proper number of chromosome pairs. E.g. a euploid human embryo has 23 pairs of chromosomes for a total of 46 chromosomes.

Most cases of aneuploidy result in termination of the developing fetus, but there can be cases of live birth. An extra or missing chromosome is a common cause of genetic disorders (birth defects). It is therefore of particular interest to be able to detect aneuploid IVF embryos before transfer, because some of these embryos have the ability to develop into live births, however possibly with unwanted genetic disorders.

*Other parameters*

Rearrangement of cellular position = Cellular movement (see below)

Cellular movement: Movement of the center of the cell and the outer cell membrane. Internal movement of organelles within the cell is NOT cellular movement. The outer cell membrane is a dynamic structure, so the cell boundary will continually change position slightly. However, these slight fluctuations are not considered cellular movement. Cellular movement is when the center of gravity for the cell and its position with respect to other cells change as well as when cells divide. Cellular movement can be quantified by calculating the difference between two consecutive digital images of the moving cell. An example of such quantification is described in detail in the pending PCT application entitled "Determination of a change in a cell population", filed Oct 16 2006. However, other methods to determine movement of the cellular center of gravity, and/or position of the cytoplasm membrane may be envisioned e.g. by using FertiMorph software (ImageHouse Medical, Copenhagen, Denmark) to semiautomatically outline the boundary of each blastomere in consecutive optical transects through an embryo.

Organelle movement: Movement of internal organelles and organelle membranes within the embryo which may be visible by microscopy. Organelle movement is not Cellular movement in the context of this application.

Movement: spatial rearrangement of objects. Movements are characterized and/or quantified and/or described by many different parameters including but restricted to: extent of movement, area and/or volume involved in movement, rotation, translation vectors, orientation of movement, speed of movement, resizing, inflation/deflation etc. Different measurements of cellular or organelle movement may thus be used for different purposes some of these reflect the extent or magnitude of movement, some the spatial distribution of moving objects, some the trajectories or volumes being afflicted by the movement.

Embryo quality is a measure of the ability of said embryo to successfully implant and develop in the uterus after transfer. Embryos of high quality have a higher probability of successfully implant and develop in the uterus after transfer than low quality embryos. However, even a high quality embryo is not a guarantee for implantation as the actual transfer and the woman's receptivity highly influences the final result.

Viability and quality are used interchangeably in this document. Embryo quality (or viability) measurement is a parameter intended to reflect the quality (or viability) of an embryo such that embryos with high values of the quality parameter have a high probability of being of high quality (or viability), and low probability of being low quality (or viability). Whereas embryos with an associated low value for the quality (or viability) parameter only have a low probability of having a high quality (or viability) and a high probability of being low quality (or viability)

**Description of Drawings**

Fig. 1: Nomenclature for the cleavage pattern of an embryo until the eight blastomere stage showing cleavage times (t2-t5), duration of cell cycles (cc1-cc3), and synchronies (s1-s3) in relation to images obtained.

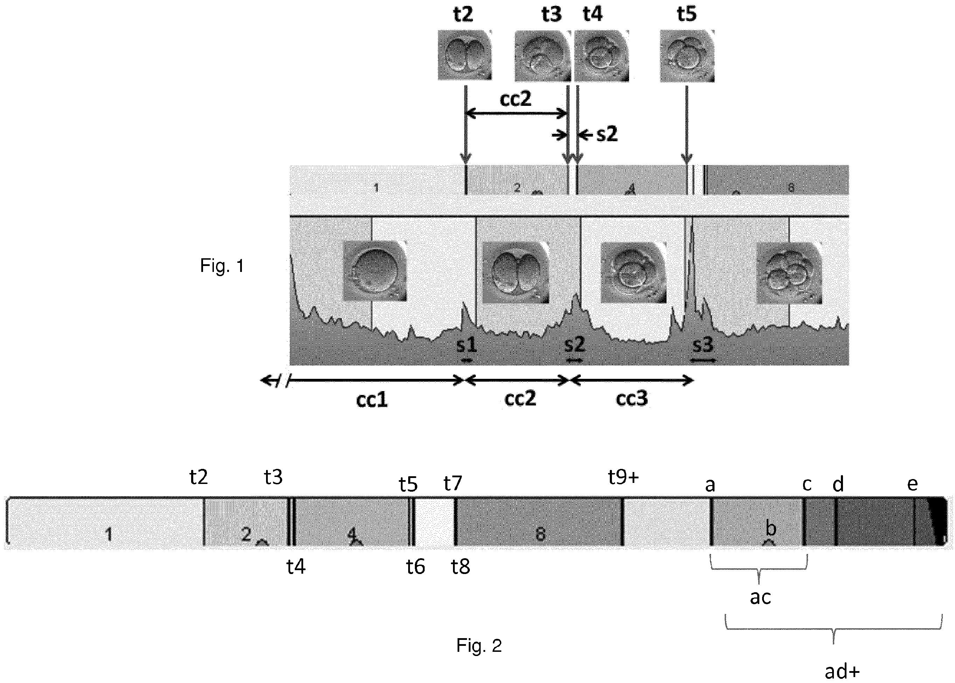

Fig. 2: The development of the embryo until the blastocyst stage. The number refers to the number of blastomeres in each stage. The letters a to e refer to the following kinetic parameters. a: Morula (M), b: Initial differentiation of trophectoderm (IDT), c: blastocyst (BI), d: Onset of expansion of the blastocyst (EB), e: Hatching Blastocyst (HB). Initial Compaction (IC) can be observed between t5 and Morula (a), if present usually IC precedes Morula by minutes to a few hours. Partial compaction (PC) can be observed between stages a and c if present. Vacuolization" (VC(X)) and contractions (NC(X)) can be observed between stages a and d+ if present.

Fig. 3a: A picture of an embryo immediately prior to initial compaction.

Fig. 3b: A picture of an embryo at the time of initial compaction. Compaction is a process wherein an intensification of the contacts between the blastomeres with tight junction and desmosomes result in reduction of the intercellular space and a blurring of the cell contours as seen in fig. 3b. When compaction is complete no plasma-membranes between any blastomeres are visible and the embryo can be defined as a morula.

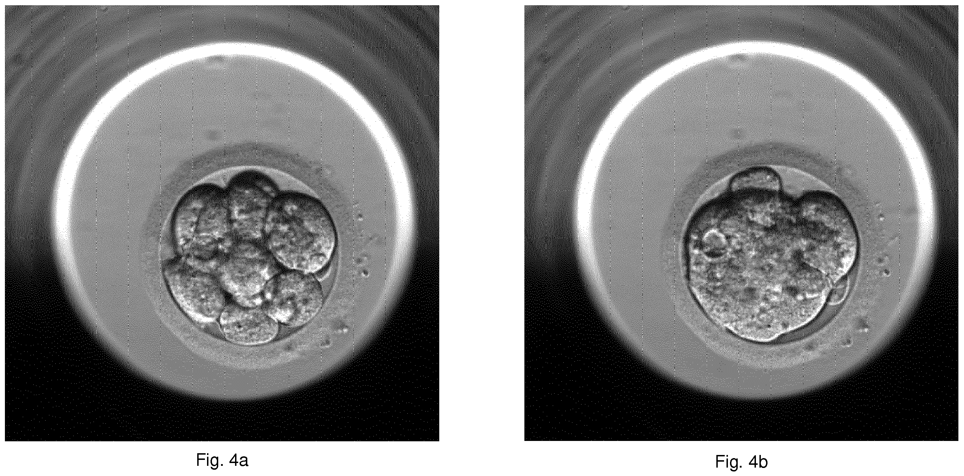

Fig. 4a: A picture of an embryo before full Morula.

Fig. 4b: Same embryo as in fig. 4a, 2.5 hours later where the embryo is in the Morula stage. When compaction is complete no plasma-membranes between any blastomeres are visible and the embryo can be defined as a morula.

Fig. 5a: A picture of an embryo immediately prior to initial differentiation of trophectoderm.

Fig. 5b: Same embryo as in fig. 5, at the time of initial differentiation of trophectoderm, which is the first time during the morula stage distinct trophectoderm cells are recognized, as indicated by the three arrows. It describes the onset of differentiation of the trophectoderm cells. The blastomeres gradually become flattened and elongate creating a barrier between the outside environment and the inner cell part of the morula.

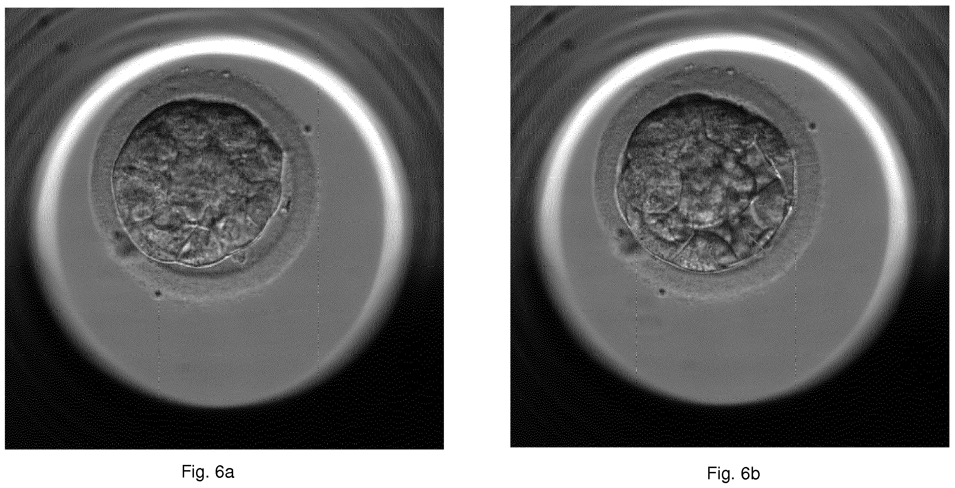

Fig. 6a: A picture of an embryo immediately prior to onset of cavitation (early blastocyst).

Fig. 6b: Same embryo as in fig. 6a at the time of onset of cavitation, which is the first time a fluid-filled cavity, the blastocoel, can be observed as indicated by the two arrows. The outer layer of the morula with contact to the outside environment begins to actively pump salt and water into the intercellular space, as a result of which a cavity (the blastocoel) begins to form.

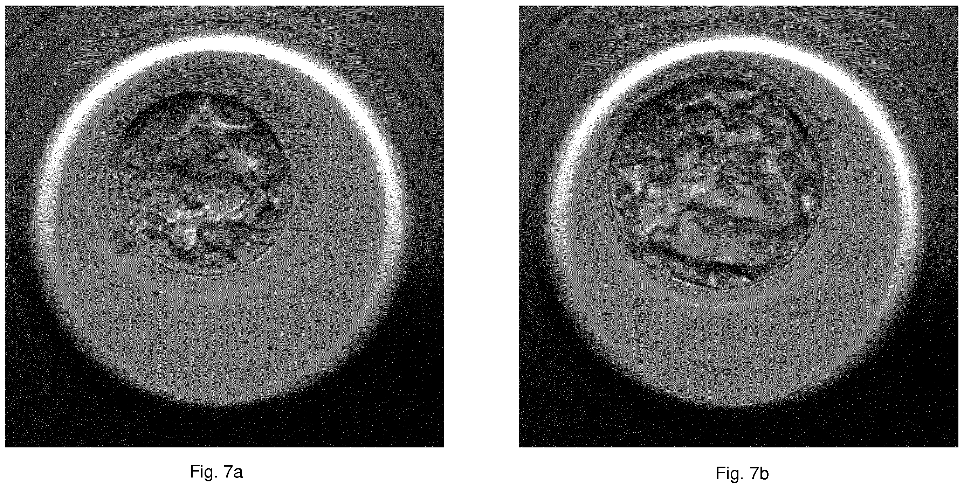

Fig. 7a: A picture of a blastocyst prior to onset of expansion of the blastocyst (EB).

Fig. 7b: Same embryo as in fig. 7a but now the blastocyst is expanding. The onset of expansion is the first time the embryo has filled out the periviteline space and starts moving/expanding Zona Pelucidae. EB describes the initiation of the embryos expansion. As the blastocyst expands the zona pellucida becomes visibly thinner.

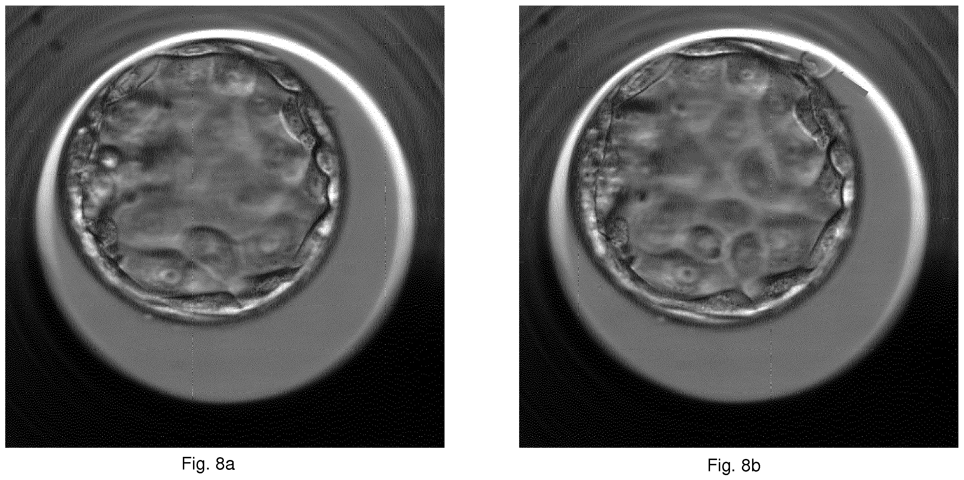

Fig. 8a: A picture of an expanded blastocyst immediately prior to hatching.

Fig. 8b: Same embryo as in fig. 8a but now the blastocyst is hatching, which is the first time a trophectoderm cell has escaped / penetrated the zona pellucida.

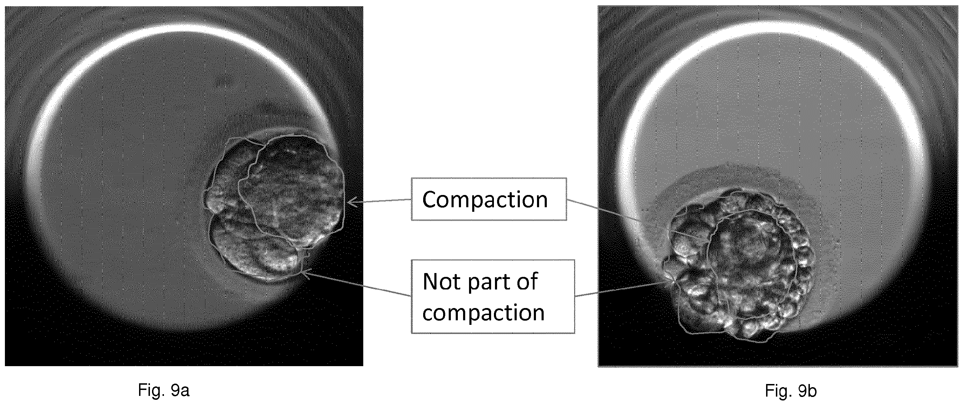

Fig. 9a and 9b are pictures of embryos with only partial compaction which is an uneven compaction process where one or more of the blastomeres are not included in the compaction, as illustrated by the markings in the figures.

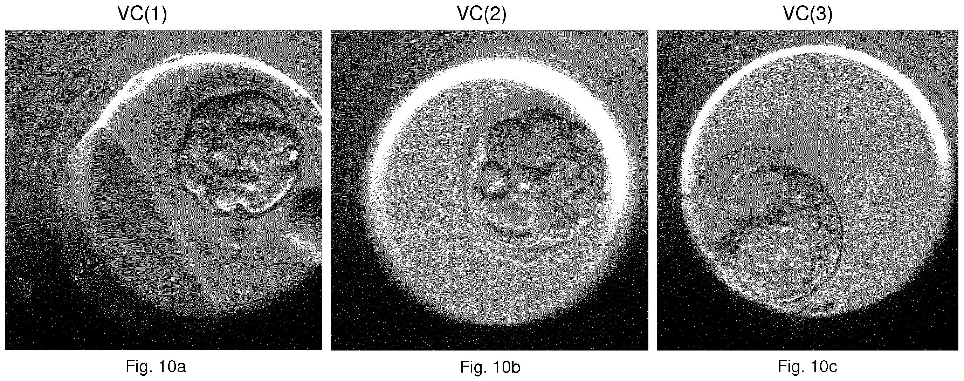

Fig. 10a,10b and 10c are pictures of embryos illustrating different degrees (1, 2 and 3) of vacuolization, which is the extent of vacuolization after the morula stage.

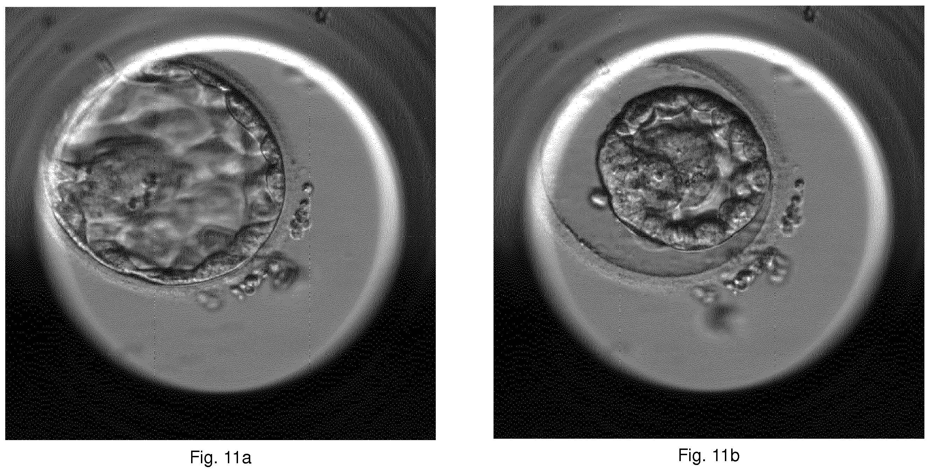

Fig. 11 a and 11b are pictures of the same embryo with twenty minutes difference. In fig. 11 a the blastocyst is expanded and twenty minutes later in fig. 11b the blastocyst is clearly visibly contracted. A contraction is defined as a reduction in the cross sectional surface area of the embryo cross section of more than 15%.

Fig. 12: Schematic hierarchical decision tree model with the parameters t5-s2-cc2 based on: i) Morphological screening; ii) absence of exclusion criteria; iii) timing of cell division to five cells (t5); iv) synchrony of divisions from 2-cell to 4-cell stage, s2, i.e. duration of 3-cell stage; v) duration of second cell cycle, cc2, i.e. time between division to 3-cell stage and division to 5-cell stage. The classification generates ten grades of embryos with increasing expected implantation potential (right to left) and almost equal number of embryos in each.

Fig. 13a and 13b: Known Implantation data (see example 1) divided into quartiles with respect to t2 and with the expected value for each quartile (fig. 13a). From these quartile groups a new target group is formed by the three neighboring quartiles Q1, Q2 and Q3, having similar probabilities (fig. 13b).

Fig. 14: Example of a decision tree model.

Fig. 15: Example of a decision tree model.

Fig. 16: Known Implantation data (see examples 1 and 3) showing 351 embryos with known outcome plotted in a graph with tEB along the first axis and tBI along the second axis (where tBI are referred to as "tB"). Successful implantations are shown with squares whereas failed implantations are shown with triangles.

**Detailed description of the invention**

**Determination of quality**

The search for prognostic factors that predict embryo development and the outcome of in vitro fertilization (IVF) treatment have attracted considerable research attention as it is anticipated that knowledge of such factors may improve future IVF treatments.

As discussed above one promising predictive factor is the precise timing of key events in early embryo development. Studies that involve imaging have been limited to measurements of early development, such as pronuclear formation and fusion, and time to first cleavage (Nagy, Z.P. 1994, Fenwick, J. 2002, Lundin, K. 2001, Lemmen, J.G. 2008). An important finding of the time-lapse analysis is a correlation between the early cleavage pattern to the 4-cell stage and subsequent development to the blastocyst stage. Morphokinetic analysis on the development of bovine embryos have also been published, where timing, duration and intervals between cell cleavages in early embryo development successfully predicted subsequent development to the expanded blastocyst stage (Ramsing 2006, Ramsing 2007).

The present inventors have performed a large clinical study involving many human embryos and monitoring the development, not only until formation of a blastocyst, but further until sign of implantation of the embryo. In this study important differences in the temporal patterns of development between the embryos that implanted (i.e. embryos that were transferred and subsequently led to successful implantation) and those that did not (i.e. embryos that were transferred but did not lead to successful implantation) were observed. By using implantation as the endpoint, not only embryo competence for blastocyst formation, but also subsequent highly essential processes such as hatching and successful implantation in the uterus is assessed.

It has been found that there exists an optimal time range for parameters characterizing the embryonic cell divisions. The observations support the hypothesis that the viability of embryos is associated with a highly regulated sequence of cellular events that begin at the time of fertilization. In this clinical study on exclusively good quality embryos, it has been confirmed that an embryo's capability to implant is correlated with numerous different cellular events. The complexity, structure and parameters in the models must be adaptable to different clinical situations like incubation temperature, transfer times, culture media and other.

Timing of early events in embryonic development correlates with development into a blastocyst, and the development into a blastocyst is a necessity for a successful implantation and thus the formation of a blastocyst is a quality parameter in itself. However it has been found that the development into a blastocyst does not necessarily correlate with successful implantation of the embryo.

Supporters of early implantation at day 2 have argued that the extended culture of embryos to the blastocyst stage at around day 5 give rise to potential risks because the culture period is significantly prolonged which may disrupt embryo integrity. However, an extended culture period to the blastocyst stage has several advantages. Cultured human embryos have an average blastocyst formation rate of only approx. 30-50%, and by extending the culture period a large part of the low quality embryos have automatically been excluded, by not forming the blastocyst. Furthermore, after EGA at around the 5-8 blastomere stage the embryos own DNA controls the development. By evaluating the embryos at the blastocyst stage high quality embryos can be identified with a higher degree of certainty.

Thus, the data allows the detection of blastocyst related developmental criteria for implantation potential. The results in particular indicate that timing of late events, such as the onset of cavitation, are a consistently good indicator of implantation potential, and that the discrimination between implanting and non-implanting embryos is improved when using blastocyst quality criteria, e.g. tBI as opposed to the earlier events (t2, t3 and t4). The presented data indicate that incubating the embryos to the blastocyst stage can give additional important information that will improve the ability to select a viable embryo with high implantation potential. The claims list a number of embryo quality criteria and blastocyst quality criteria that may be applied singly or combined in groups to assess embryo quality.

One embodiment of the invention relates to a method for determining embryo quality comprising monitoring the embryo for a time period and determining one or more blastocyst quality criteria for said embryo, wherein said time period comprises the time from fertilization to a blastocyst stage, and wherein 1) the duration of a first time period from fertilization until translation of maternally inherited mRNA in the blastomeres is completed and 2) the duration of a second time period from initiation of transcription of the blastomeres own DNA to said blastocyst stage are determined, and wherein a blastocyst quality criterion is the ratio of said first and second time periods, and based on said one or more blastocyst quality criteria determining the embryo quality.

A further embodiment of the invention relates to a method for determining embryo quality comprising monitoring the embryo for a time period, said time period comprises the time from fertilization to a blastocyst stage, wherein 1) the duration of a first time period from fertilization to a 5 blastomere embryo and 2) the duration of a second time period from the 5 blastomere embryo to said blastocyst stage are determined, and wherein a blastocyst quality criterion is the ratio of said first and second time periods, and based on said blastocyst quality criterion determining the embryo quality.

The time from fertilization to the blastocyst stage is thereby divided into two time periods and the ratio between these time periods is a blastocyst quality criterion. The reason for dividing at the 5 blastomere stage is that this is approx. the time of embryonic gene activation. Thus, in a further embodiment of the invention the time period comprises the time from fertilization to a blastocyst stage, wherein 1) the duration of a first time period from fertilization until translation of maternally inherited mRNA in the blastomeres is completed and 2) the duration of a second time period from initiation of transcription of the blastomeres own DNA to said blastocyst stage are determined, and wherein a blastocyst quality criterion is the ratio of said first and second time periods.

In a further embodiment of the invention the ratio of the second time period divided by the first time period is an indicator of high embryo quality if said ratio is greater than a predefined value.

A corresponding blastocyst quality criterion can be provided by determining 1) the duration of a first time period from fertilization to blastocyst, and 2) the duration of a second time period from initiation of transcription of the blastomeres own DNA to said blastocyst stage and taking the ratio of these time periods. This ratio provides information on how much of the total time period from fertilization to blastocyst the embryo's own DNA is in control. Again, the ratio of the second time period divided by the first time period is an indicator of high embryo quality if said ratio is greater than a predefined value. This ratio can be seen as a measure for the relative development speed in a certain period relative to the overall development speed until that stage. Thus, it seems that the embryos that take more time to develop from the time where the embryos own genome takes over at EGA relative to the overall development time has a higher probability to implant. Or similarly: The embryos that take more time to develop from the time where the embryos own genome takes over at EGA relative to the time before EGA when the maternally inherited mRNA is in control, has a higher probability to implant.

Early cleavage (i.e. low t2) has long been known as an embryo quality indicator. Low t4 and t5 are also quality indicators, thereby showing that fast development before EGA is an indicator of high quality. Herein it is also demonstrated that embryos that reach the blastocyst stage (Blastocyst, tBl) before approx. 96 hours have a higher probability for implantation, thereby showing that fast development in general all the way to the blastocyst stage is a quality indicator. It is therefore surprising that a slow phase in this fast development, i.e. the phase after EGA, is a quality indicator. Typically there is no clear-cut determination of the time of EGA and it may be defined at e.g. t4, t5, or t8. It may also be defined that "before EGA" is the development until the 4 blastomere stage, i.e. t4, and "after EGA" may be defined as after the 8 blastomere stage.

The abovementioned blastocyst stage may be selected from the group of: initial compaction (IC), Morula (M), initial differentiation of trophectoderm cells (IDT), early blastocyst (ERB), blastocyst (Bl), expansion of blastocyst (EB), first contraction (CPS(1)), second contraction (CPS(2)), third contraction (CPS(3)), fourth contraction (CPS(4)), fifth contraction (CPS(5)), sixth contraction (CPS(6)), seventh contraction (CPS(7)), hatching blastocyst (HB), and fully hatched blastocyst (FH). Thus, tIC is the time from fertilization to initial compaction, tM is the time from fertilization to Morula, etc.

A further blastocyst quality criterion may be the determination of the absolute or relative 2D and/or 3D expansion of the blastocyst, e.g. the speed of the blastocoel expansion, where e.g. a quick expansion may be a quality indicator. A further blastocyst quality criterion may be the largest degree of expansion of the blastocyst, e.g. the diameter prior to expansion relative to the largest embryo diameter for the expanded blastocyst. Thus, a blastocyst quality criterion may be the determination of the diameter and/or the volume of the embryo at the onset of expansion. Further, a blastocyst quality criterion may be the determination of the maximum diameter and/or the maximum volume of the blastocyst before hatching.

*Multiple variables*

Multiple variables may be used when choosing selection criteria. When using multiple variables it can be an advantage that the variables are selected progressively such that initially one or more of the variables that can be determined early with a high accuracy are chosen, e.g. t2, t3, t4 or t5. Later other variables that can be more difficult to determine and is associated with a higher uncertainty can be used.

*Normalized or relative parameters*

In one embodiment of the invention an embryo quality criterion is selected from the group of normalized morphological embryo parameters, e.g. the group of normalized morphological parameters based on two, three, four, five or more parameters selected from the group of t2, t3, t4, t5, t6, t7 and t8. By normalizing the parameters the time of fertilization may be "removed" from the embryo quality assessment. Further, a normalized morphological embryo parameter may better describe the uniformity and/or regularity of the developmental rate of a specific embryo independent of the environmental conditions, because instead of comparing to "globally" determined absolute time intervals that may depend on the local environmental conditions, the use of normalized parameters ensure that specific ratios of time intervals can be compared to "globally" determined normalized parameters, thereby providing additional information of the embryo development.

*Exclusion criteria*

An embryo population may be subject to one or more exclusion criteria in order to exclude embryos from the population with a low probability of implantation success, i.e. the outliers. This may be embryos that fulfil many of the positive selection criteria but show unusual behaviour in just one or two selection criteria. Examples of exclusion criteria are number of contractions of the blastocyst, the degree of vacuolization and uneven compaction. However, exclusion criteria may also be applied to the morphological embryo parameters. It has long been known that slowly developing embryos are an indication of poor quality, reflected in a very high value of t2 (>31.8 hours), but cleavage from one blastomere directly to three blastomeres may also be an indication of a poor quality embryo associated with low implantation rate despite of a fast t3.

A specific exclusion criterion pointing out a group of embryos in a population with a low probability of implantation does not imply that the rest of the population has a high probability of implantation. An exclusion criterion only indicates poor quality embryos. Thus, in one embodiment of the invention said one or more blastocyst quality criteria are combined with one or more exclusion criteria.

*Monitoring*

The embryo is monitored regularly to obtain the relevant information, preferably at least once per hour, such as at least twice per hour, such as at least three times per hour. The monitoring is preferably conducted while the embryo is situated in the incubator used for culturing the embryo. This is preferably carried out through image acquisition of the embryo, such as discussed below in relation to time-lapse methods.

Determination of selection criteria's can be done for example by visual inspection of the images of the embryo and/or by automated methods such as described in detail in the pending PCT application entitled "Determination of a change in a cell population" filed Oct 16 2006. Furthermore, other methods to determine selection criteria's can be done by determining the position of the cytoplasm membrane by envisioned e.g. by using FertiMorph software (ImageHouse Medicall Copenhagen, Denmark). The described methods can be used alone or in combination with visual inspection of the images of the embryo and/or with automated methods as described above.

*Decision tree model*

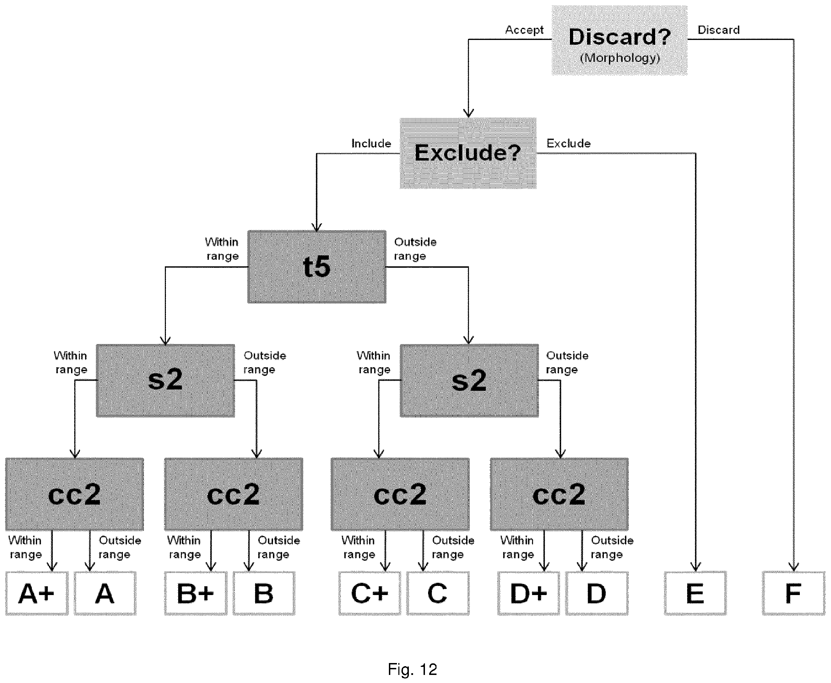

In particularly, the criteria may be combined in a hierarchical form, as shown in Figs. 12, 14 and 15 (see also example 1 for more information) thereby giving rise to a decision tree model (or classification tree model) to select embryos with higher implantation probabilities. In a classification tree model several variables are used to split the embryos into groups with different associated probability of implantation success rate by using successive splitting rules. The classification tree model can be optimized under a set of given constraints selecting the optimal variables to use in the splitting rules from a set of possible variables. The variables used in the model can e.g. be morphological embryo parameters based on time intervals between morphological events and the corresponding normalized morphological embryo parameters and discrete variables (e.g. multi nuclearity or evenness of blastomeres), or any combination of these variables. This type of models can be evaluated using area under the ROC curve (AUC). AUC is 0.5 if no splitting is applied and the splitting improves the predictive power if AUC > 0.5.

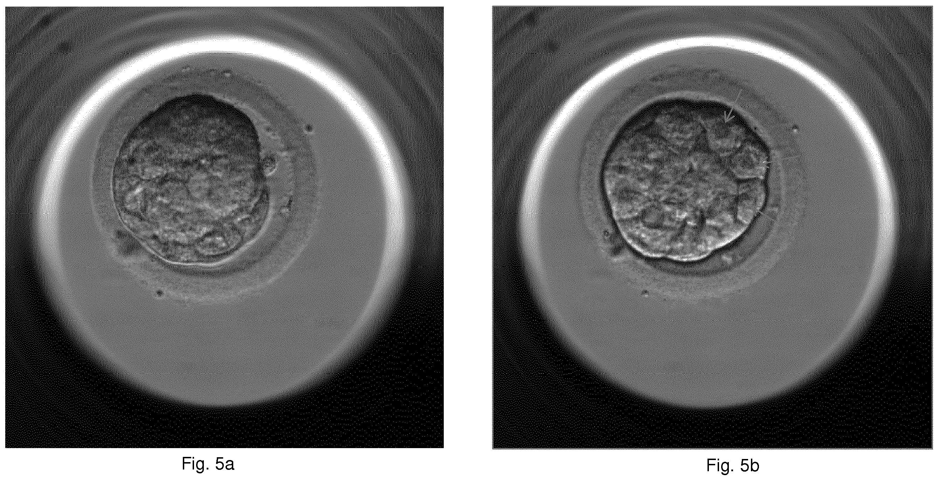

In the decision tree depicted in fig. 12 embryos are subdivided into 6 categories from A to F. Four of these categories (A to D) were further subdivided into two sub-categories (+) or (-) as shown in Figure 5, giving a total of 10 categories. The hierarchical decision procedure start with a morphological screening of all embryos in a cohort to eliminate those embryos that are clearly NOT viable (i.e. highly abnormal, attretic or clearly arrested embryos). Those embryos that are clearly not viable are discarded and not considered for transfer (category F). Next step in the model is to exclude embryos that fulfil any of the three exclusion criteria: i) uneven blastomere size at the 2 cell stage, ii) abrupt division from one to three or more cells; or iii) multi-nucleation at the four cell stage (category E). The subsequent levels in the model follow a strict hierarchy based on the binary timing variables t5, s2 and cc2. First, if the value of t5 falls inside the optimal range the embryo is categorized as A or B. If the value of t5 falls outside the optimal range (or if t5 has not yet been observed at 64 hours) the embryo is categorized as C or D.

If the value of s2 falls inside the optimal range the embryo is categorized as A or C depending on t5 and similarly if the value of s2 falls outside the optimal range the embryo is categorized as B or D depending on t5.

Finally, the embryo is categorized with the extra plus (+) if the value for cc2 is inside the optimal range (A+/B+/C+/D+) and is categorized as A,B,C,D if the value for cc2 is outside the optimal range.

The decision tree models can be evaluated using receiver operator characteristic (ROC) methods evaluated by multi-class AUC. Multiclass AUC expresses how well the model sorts the embryos with respect to probability for implanting. AUC lies between 0.5 and 1 where 0.5 is the sorting power of a random model (no effect of the model) and a higher AUC indicate a better sorting compared to the random model.

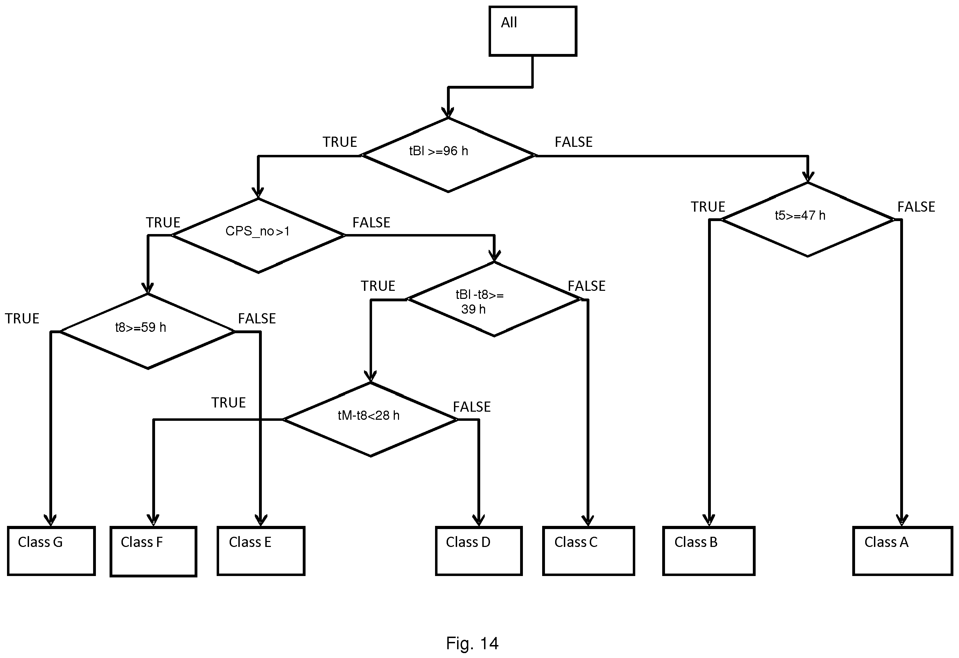

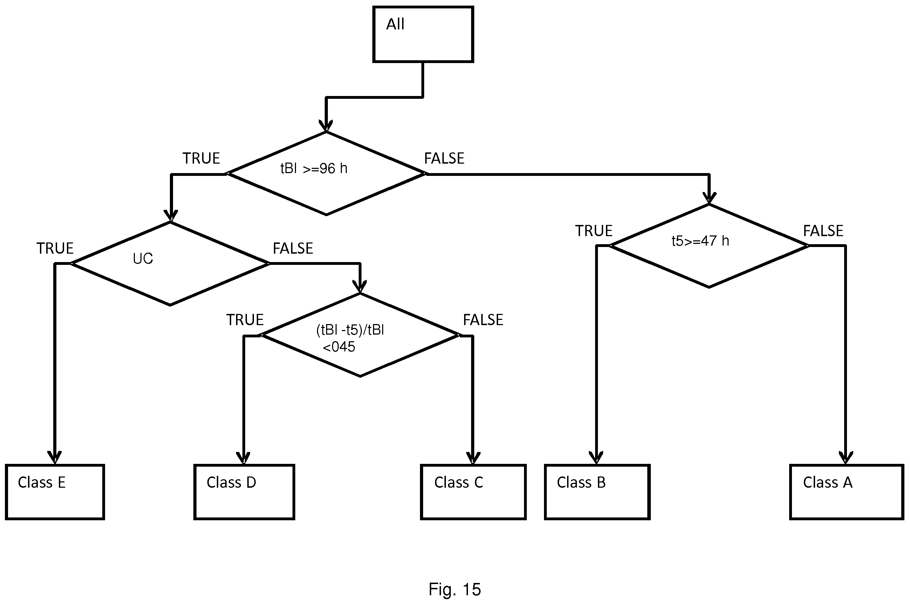

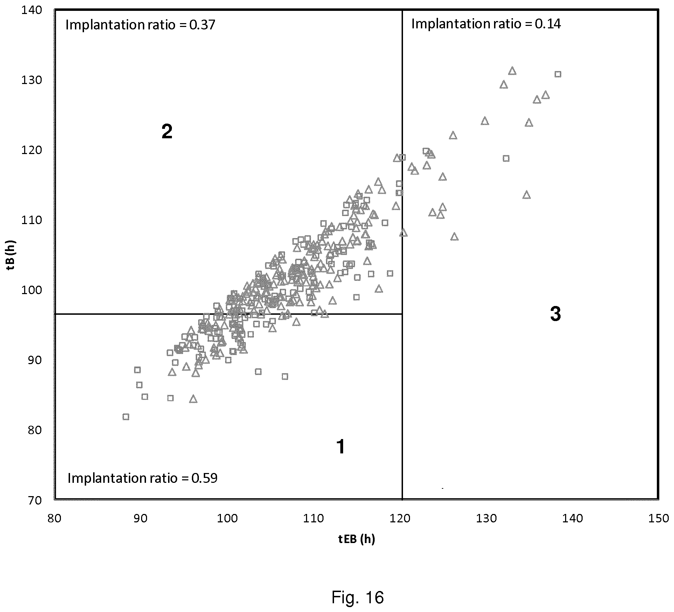

Decision tree models have been constructed based on KID data from 407 human embryos (see example 1) and the multiclass AUC have been determined. The two decision trees in figs. 14 and 15 are based on embryo quality criteria, blastocyst quality criteria and exclusion criteria. For fig. 14 multiclass AUC = 0.68, whereas AUC is slightly lower at 0.64 for the decision tree in fig. 15. By means of these decision tree models the 407 embryos have been classified into seven quality classes A-G with decreasing implantation probability (fig. 15) and into five classes A-E (fig. 16).

The probability of implantation of a specific embryo from a specific woman depends on many other parameters. However, this dataset provides a unique opportunity to test the quality and exclusion criteria presented herein in order to optimize the classification of IVF embryos. E.g. to classify (in terms of quality) a number of embryos taken from a single woman in order to select the best embryo(s) for transfer. Possibly none of the embryos from a single woman fulfils all optimal quality criteria because all embryos are mediocre or poor quality. However, a transfer must be performed and a classification of the embryos is therefore important to select the best of the embryos. Thus, the highest possible AUC is naturally preferred but within the field of embryo selection any improvement in sorting compared to the random model is good and can be considered to improve the selection of good embryos.

*Logistic regression model*

The criteria may also be combined in form of a logistic regression model that predicts the odds of implantation success of the embryo (se example 2).The model can be affected by both discrete and continuous variables. The continuous variables used shall have a monotone effect on the odds (either increasing with increasing value of the variable or decreasing with the value of the variable).

*Combination with measurements of movement*

The quality criteria discussed above may also be combined with determinations of movement of the embryo, such as i) determining the extent and/or spatial distribution of cellular or organelle movement during the cell cleavage period; and/or ii) determining the extent and/or spatial distribution of cellular or organelle movement during the inter-cleavage period thereby obtaining an embryo quality measure.

Volumes within the zona pellucida that are devoid of movement (or similarly areas in a projected 2D image of the embryo that remain stationary) are an indication of "dead" zones within the embryo. The more and larger these immotile "dead" zones the lower the probability of successful embryo development. Large areas within a time-lapse series of embryo images without any type of movement (i.e. neither cellular nor organelle movement) indicates low viability. Organelle movement should generally be detectable in the entire embryo even when only comparing two or a few consecutive frames. Cellular movement may be more localized especially in the later phases of embryo development.

The cell positions are usually relatively stationary between cell cleavages (i.e. little cellular movement), except for a short time interval around each cell cleavage, where the cleavage of one cell into two leads to brief but considerable rearrangement of the dividing cells as well as the surrounding cells (i.e. pronounced cellular movement). The lesser movement between cleavages is preferred.

In one embodiment, in order to determine movement relating to either cleavage and inter-cleavage periods, the length of each cleavage period may be determined as well as the length of each inter-cleavage period. Preferably the period of cellular movement in at least two inter-cleavage periods is determined as well as the extent of cellular movement in at least two inter-cleavage periods. Furthermore, it has been found that rapid cleavage seems to increase quality of the embryo, where rapid normally means less than 2 hours.

In relation to movement during cleavage and inter-cleavage periods we also refer to PCT application WO 2007/144001.

A neural network or other quantitative pattern recognition algorithms may be used to evaluate the complex cell motility patterns described above, for example using different mathematical models (linear, Princepal component analysis, Markov models etc.)

*Time-lapse monitoring*

A particular use of the invention is to evaluate image series of developing embryos (time-lapse images). These time-lapse images may be analyzed by difference imaging equipment (see for example WO 2007/042044 entitled "Determination of a change in a cell population"). The resulting difference images can be used to quantify the amount of change occurring between consecutive frames in an image series.

The invention may be applied to analysis of difference image data, where the changing positions of the cell boundaries (i.e. cell membranes) as a consequence of cellular movement causes a range parameters derived from the difference image to rise temporarily (see WO 2007/042044). These parameters include (but are not restricted to) a rise in the mean absolute intensity or variance. Cell cleavages and their duration and related cellular re-arrangement can thus be detected by temporary change, an increase or a decrease, in standard deviation for all pixels in the difference image or any other of the derived parameters for "blastomere activity" listed in WO 2007/042044. However the selection criteria may also be applied to visual observations and analysis of time-lapse images and other temporally resolved data (e.g. excretion or uptake of metabolites, changes in physical or chemical appearance, diffraction, scatter, absorption etc.) related to embryo.

Of particular interest are the onset, magnitude and duration of cell cleavages that may be quantified as peaks or valleys, in derived parameter values. These extremes, peaks or valleys, frequently denote cell cleavage events. The shape of each peak also provides additional information as may the size of the peak in general. A peak may also denote an abrupt collapse of a blastomere and concurrent cell death. However, it may be possible to separate cell cleavage events and cell death events by the peak shape and change in base values before and after the event. The baseline of most parameters is usually not affected by cell cleavage whereas cell lysis is frequently accompanied by a marked change in the baseline value (for most parameters in a decrease following lysis.)

In summary, the present invention demonstrates that routine time-lapse monitoring of embryo development in a clinical setting (i.e. automatic image acquisition in an undisturbed controlled incubation environment) provide novel information about developmental parameters that differ between implanting and non-implanting embryos.

**Embryo**

In some cases the term "embryo" is used to describe a fertilized oocyte after implantation in the uterus until 8 weeks after fertilization at which stage it becomes a foetus. According to this definition the fertilized oocyte is often called a pre-embryo until implantation occurs. However, throughout this patent application we will use a broader definition of the term embryo, which includes the pre-embryo phase. It thus encompasses all developmental stages from the fertilization of the oocyte through morula, blastocyst stages hatching and implantation.

An embryo is approximately spherical and is composed of one or more cells (blastomeres) surrounded by a gelatine-like shell, the acellular matrix known as the zona pellucida. The zona pellucida performs a variety of functions until the embryo hatches, and is a good landmark for embryo evaluation. The zona pellucida is spherical and translucent, and should be clearly distinguishable from cellular debris.

An embryo is formed when an oocyte is fertilized by fusion or injection of a sperm cell (spermatozoa). The term is traditionally used also after hatching (i.e. rupture of zona pelucida) and the ensuing implantation. For humans the fertilized oocyte is traditionally called an embryo for the first 8 weeks. After that (i.e. after eight weeks and when all major organs have been formed) it is called a foetus. However the distinction between embryo and foetus is not generally well defined.

Accordingly, the term embryo is used in the following to denote each of the stages fertilized oocyte, zygote, 2-cell, 4-cell, 8-cell, 16-cell, morula, blastocyst, expanded blastocyst and hatched blastocyst, as well as all stages in between (e.g. 3 -cell or 5-cell)

**Other measurements**

A final analysis step could include a comparison of the made observations with similar observations of embryos of different quality and development competence, as well as comparing parameter values for a given embryo with other quantitative measurements made on the same embryo. This may include a comparison with online measurements such as blastomere motility, respiration rate, amino acid uptake etc. A combined dataset of blastomere motility analysis, respiration rates and other quantitative parameters are likely to improve embryo selection and reliably enable embryologist to choose the best embryos for transfer.

Thus, in one embodiment the method according to the invention may be combined with other measurements in order to evaluate the embryo in question, and may be used for selection of competent embryos for transfer to the recipient.

Such other measurements may be selected from the group of respiration rate, amino acid uptake, motility analysis, blastomere motility, morphology, blastomere size, blastomere granulation, fragmentation, blastomere colour, polar body orientation, nucleation, spindle formation and integrity, and numerous other qualitative measurements. The respiration measurement may be conducted as described in PCT publication no. WO 2004/056265.

**Culture medium**

In a preferred embodiment the observations are conducted during cultivation of the cell population, such as wherein the cell population is positioned in a culture medium. Means for culturing cell population are known in the art. An example of culturing an embryo is described in PCT publication no. WO 2004/056265.

**Data carrier**

The invention further relates to a data carrier comprising a computer program directly loadable in the memory of a digital processing device and comprising computer code portions constituting means for executing the method of the invention as described above.
The data carrier may be a magnetic or optical disk or in the shape of an electronic card as for example the type EEPROM or Flash, and designed to be loaded into existing digital processing means.

**Selection or identification of embryos**

The present invention further provides a method for selecting an embryo for transplantation. The method implies that the embryo has been monitored as discussed above to determine when cell cleavages have occurred.
The selection or identifying method may be combined with other measurements as described above in order to evaluate the quality of the embryo. The important criteria in a morphological evaluation of embryos are: (1) shape of the embryo including number of blastomeres and degree of fragmentation; (2) presence and quality of a zona pellucida; (3) size; (4) colour and texture; (5) knowledge of the age of the embryo in relation to its developmental stage, and (6) blastomere membrane integrity.
The transplantation may then be conducted by any suitable method known to the skilled person.

**Example 1 - data analysis based on known implantation data**

This analysis is based on known implantation data (KID) of 407 embryos incubated under different conditions (patient characteristics, clinical practices and rules and regulations). The KID embryos are all transferred embryos with known implantation. With multiple embryo transfers only total failure of implantation or total success is used. All multiple transfers with implantation that have less implanted embryos than transferred were discarded to enable the implantation success for the specific embryo. The implantation success takes the value 1 if the transferred embryo led to successful implantation implanted and 0 if not. The number of embryos (N) used for calculating the expected value (probability of success) of the target and non-target groups is different for different variables.

*Single variable*

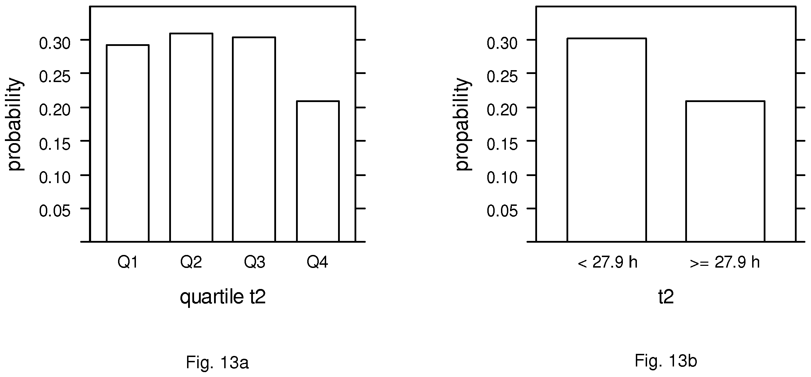

The data were divided into quartiles with respect to a single continuous variable (e.g. t5) and the expected value (probability of getting a success with one trial) of each quartile was calculated. From these quartile groups a new group was formed (the target group) either by the quartile with the highest expected value or by two or three neighboring quartiles having similar probability (see example in figures 13a and 13b). A Fisher's exact test was used to test the hypothesis that the probability of implanting (expected value of the KID data) of embryos in the target group and outside the group was equal. The hypothesis was rejected if the p-value was < 0.1 indicating that there was a difference between groups, and otherwise considered non-significant.
Odds ratio (OR) of two groups with associated probabilities ($p_i$ and $p_j$) is calculated as

$$OR_{ij} = \frac{\dfrac{p_i}{1-p_i}}{\dfrac{p_j}{1-p_j}}$$

The odds of the first group is $\frac{p_i}{1-p_i}$ and the odds of the second group is $\frac{p_j}{1-p_j}$. The odds ratio for implantation of two groups can be tested using the Fishers test providing the p-value. The odds ratio provides the "odds" for being inside the target group. A high odds ratio for a target group for a specific parameter is better. With H0 odds ratio is 1.

Table 1: Continuous blastocyst variables used in the analysis

| Variable | Description |
|---|---|
| tIC | Time from fertilization to initial compaction |
| tM | Time from fertilization to morula |
| tIDT | Time from fertilization to initial differentiation of trophectoderm cells |
| tERB | Time from fertilization to early blastocyct |
| tBI | Time from fertilization to blastocyst |
| tEB | Time from fertilization to expansion of blastocyst |
| tCPS(1) | Time from fertilization to first contraction |
| tCPS(2) | Time from fertilization to second contraction |
| tCPS(3) | Time from fertilization to third contraction |
| tHB | Time from fertilization to hatching |
| tFH | Time from fertilization to fully hatched |

Table 2: Discrete blastocyst variables used in the analysis

| Variable | Description |
|---|---|
| sixty_hours_cells | Number of blastomeres after 60 hours |
| cells_before_M | Number of cells before morula (taking the values 4,5,6,7,8 and 9+) |
| CPS_no | Number of contractions of the blastocyst |
| VC_grade | Vacuolization (taking the values 0,1,2,3), 0 is no registered vacuolization |
| UC | Uneven compaction (taking the values TRUE, FALSE) |

Table 3: Quartile analysis of the time variables with the target group, the quartiles inside and outside the target group, the target group interval, the odds ratio (inside to outside) of the target group and p-value of the Fishers test.

| Variable | Quartiles in target group | Inside target group (i) | Odds ratio ($OR_{ij}$) | p-value Fishers test |
|---|---|---|---|---|
| tIC | Q2 | 72.4-79.0 h | 2.05 | 0.08 |
| tM | Q1,Q2 | < 85.6 h | 1.68 | 0.014 |
| tIDT | Q1 | < 88.9 h | 1.89 | 0.007 |
| tERB | (Q2) | | | ns |
| tBI | Q1 | < 96.3 h | 2.62 | < 0.0001 |
| tEB | Q1 | < 101.3 h | 1.97 | 0.008 |
| tCPS(1) | Q1 | < 104.0 h | 1.90 | 0.03 |
| tHB | Q1 | < 109.5 h | 2.16 | 0.09 |

For nearly all time variables the first quartile has the highest implantation rate, indicating that fast embryo development to the blastocyst stage is indicative for high implantation rate. Only with morula this is different since there is no significant difference between the first and the second quartile with regard to tM. OR is highest with tBI and also with the most significant rejection of the H0 hypothesis. This may be related both to the fact that this is an important morphological characteristic, but it may also reflect that the stage is easy to determine from time-lapse images.

Table 4: Quartile analysis of the quality variables with the target group, the number of embryos inside and outside the target group, the odds ratio (inside to outside) of the target group and p-value of the Fishers test. Values in brackets are non-significant (ns).

| Variable | Target group (i) | n (inside/outside target group) | Odds ratio ($OR_{ij}$) | p-value Fishers test |
|---|---|---|---|---|
| Sixty_hours_cells | (9+) | | | ns |
| CPS_no (0,1,2,3) | < 3 | 282/125 | 3.33 | 0.006 |
| VC_grade (0,1,2,3) | < 2 | 383/24 | 3.35 | 0.03 |
| UC (TRUE/FALSE) | FALSE | 373/34 | 3.20 | 0.009 |
| cells_before_M (4,5,6,7,8,9+) | (9+) | | | ns |
| ERB | (FALSE) | | | ns |

From table 4 it is seen that the number of contractions should not be more than two. And that the degree of vacuolization should not be more than one. The uneven compaction is also significant. These three criteria are suitable for excluding blastocysts that have a relatively low implantation rate (OR >3), i.e. they are suitable exclusion criteria.

Table 5: Quartile analysis of the time period variables with the target group, the quartiles inside and outside the target group, the target group interval, the odds ratio (inside to outside) of the target group and p-value of the Fishers test.

| Variable | Quartiles in target group | Inside target group (i) | Odds ratio ($OR_{ij}$) | p-value Fishers test |
|---|---|---|---|---|
| tM-t5 | - | | | ns |
| tBI-t5 | (Q1,Q2,Q3) | | | ns |
| tEB-t5 | Q3 | | | ns |
| tM-t8 | Q3,Q4 | >= 27.3 h | 1.47 | 0.07 |
| tBI-t8 | (Q1,Q2) | | | ns |
| tEB-t8 | - | | | ns |
| tBI-tM | (Q1,Q2) | | | ns |
| tEB-tBI | - | | | ns |
| tHE-tEB | - | | | ns |
| tEB-tM | - | | | ns |
| tBI-tCPS(1) | (Q1,Q4) | | | ns |
| tBI-tCPS(2) | Q2,Q3,Q4 | >=10.4 h | 2.14 | 0.09 |
| tEB-tCPS(1) | Q2 | -3.3 - 2.2 h | 2.21 | 0.013 |
| tEB-tCPS(2) | (Q2) | | | ns |
| tCPS(2)-tCPS(1) | Q3,Q4 | >=7.76 | 2.56 | 0.017 |
| tCPS(3)- tCPS(2) | - | | | ns |
| max(S3a,S3b, S3c) | (Q1) | | | ns |

There is a slightly significantly lower implantation rate with a low time period between the 8 cell stage and the morula stage. This might be related to media change. The time from blastocyst to the second contraction (if this is occurring) needs to be long. Also the difference between the first two contractions should be long. This supports that fewer and later contractions may be used as quality indicator.

Table 6: Quartile analysis of normalized/relative time period variables with the target group, the quartiles inside and outside the target group, the target group interval, the odds ratio (inside to outside) of the target group and p-value of the Fishers test.

| Variable | Quartiles in target group | Inside target group (i) | Odds ratio $(OR_{ij})$ | p-value Fishers test |
|---|---|---|---|---|
| (tM-t5)/tM | (Q3,Q4) | | | ns |
| (tBI-t5)/tBI | (Q2,Q3,Q4) | | | ns |
| (tEB-t5)/tEB | Q3,Q4 | >=0.52 | 1.63 | 0.03 |
| (tM-t5)/t5 | (Q3,Q4) | | | ns |
| (tBI-t5)/t5 | (Q2,Q3,Q4) | | | ns |
| (tEB-t5)/t5 | Q3,Q4 | >=1.08 | 1.63 | 0.03 |
| (tM-t8)/tM | (Q4) | | | ns |
| (tBI-t8)/tBI | (Q3,Q4) | | | ns |
| (tEB-t8)/tEB | (Q4) | | | ns |
| (tM-t8)/t8 | (Q4) | | | ns |
| (tBI-t8)/t8 | (Q3,Q4) | | | ns |
| (tEB-t8)/t8 | (Q4) | | | ns |
| (tM-t8)/t4 | Q4 | >=0.90 | 1.55 | 0.07 |
| (tB-t8)/t4 | (Q4) | | | ns |
| (tEB-t8)/t4 | (Q4) | | | ns |
| (tBI-tM)/tBI | (Q2) | | | ns |
| (tEB-tBI)/tEB | (Q1,Q4) | | | ns |
| (tEB-tM)/tEB | (Q1,Q2,Q4) | | | ns |
| (tCPS(2)-tCPS(2))/tCPS(2) | (Q3,Q4) | | | ns |
| max(S3a,S3b, S3c))/S3 | (Q1) | | | ns |

The relative variables that include variables from both the early part of the embryo development and blastocyst variables all have a tendency towards a higher probability in the groups with a high value of the relative variables. The relative variables with only differences between blastocysts stages have less clear indications. However, please note that these variables are only based on data from 407 KID embryos.

**Example 2 - Logistic regression model**

Logistic regression is commonly used to establish models that describe the effect of continuous variables (e.g. tBI) and discrete variables (e.g. UC) on a binomial outcome (e.g. KID_value (implantation/no implantation)). The model fits the log transformed odds ($p_i/(1-p_i)$) to a linear combination of continuous and discrete variables Xj.

$$ln\left(\frac{p_i}{(1-p_i)}\right) = \beta_0 + \sum_{all\ j} \beta_{i,j} X_{i,j} + \varepsilon \Rightarrow$$

$$\frac{p_i}{(1-p_i)} = e^{\beta_0} \cdot \prod_{all\ j} e^{\beta_{i,j} X_{i,j}} \cdot e^{\varepsilon}$$

Where $p_i$ is the probability of observation $i$, $X_{i,j}$ is the value of the $j$th variable on the ith observation, $\beta 0$ is the intercept parameter, $\beta_j$ is the slope parameter and $\varepsilon$ is a random error. The model is multiplicative and exponential. A

negative value of $\beta_j$ means that a continuous variable has a decreasing effect on the model output with increasing values of the variable. With discrete variables the different values of the variable has different associated $\beta_j$ and if the variable takes that value the value of Xj is 1.

Two examples of logistic regression models describing the data are presented herein. The first model includes only variables that can be observed in the blastocysts stage and the second includes also earlier characteristics

The Akaike information criterion (AIC) was used to evaluate the relative goodness of fit of the model and to choose which variables to include in the model. AIC is a measure of both model accuracy and model complexity. If AIC increased when including an effect of a variable in the model this effect was not included.

$$\text{AIC} = 2\text{k} - 2\ln(\text{L})$$

Where k is the number of estimated parameters and L is the likelihood of the model.

Table 7: Logistic regression model A: Estimated parameters with standard error and significance for a logistic regression model with dependent variable KID_value and independent continuous variable tBI and independent discrete variable CPS_no. Akaike information criteria (AIC) for the reduced model excluding the variable in question is also shown. Multiclass AUC for the model is 0.63.

|  | Estimate | Std. Error | z value | Pr(>\|z\|) |  | AIC of reduced model (AIC full model 528) |
|---|---|---|---|---|---|---|
| (Intercept) | 5.2 | 1.5 | 3.5 | 0.0004 | *** |  |
| tBI | -0.05 | 0.014 | -3.7 | 0.0002 | *** | 538 |
| factor(CPS_no) |  |  |  |  |  | 533 |
| 1 | -0.3 | 0.3 | -1.0 | 0.3 | ns |  |
| 2 | -0.5 | 0.3 | -1.8 | 0.07 | . |  |
| 3 | -1.6 | 0.5 | -3.2 | 0.0012 | ** |  |
| UC |  |  |  |  |  | 529 |
| TRUE | -0.8 | 0.5 | -1.6 | 0.11 | ns |  |

The LRmodel A has effect of tBI (siginicant), the number of contractions (three contractions significantly different from no contractions) and uneven compaction (almost significant). Including also variables from the earlier stages result in LRmodel B with an effect of tBI (significant), the number of contractions (2 and 3 significantly different from no contractions) and there is a slightly significant effect of multinuclearity at the four cell stage and of uneven compaction.

Table 8: Logistic regression model B: Estimated parameters with standard error and significance for a logistic regression model with dependent variable KID_value and independent continuous variable tBI and independent discrete variable CPS_no and MN4_full (multinuclearity at the four cell stage). Akaike information criteria (AIC) for the reduced model excluding the variable in question are also shown. Multiclass AUC for the model is 0.65.

|  | Estimate | Std. Error | z value | Pr(>\|z\|) |  | AIC of reduced model (AIC full model 526) |
|---|---|---|---|---|---|---|
| (Intercept) | 5.3 | 1.5 | 3.6 | 0.0003 | *** |  |
| tBI | -0.052 | 0.014 | -3.7 | 0.0002 | *** | 536 |
| factor(MN4_full) |  |  |  |  |  | 528 |
| TRUE | -0.9 | 0.5 | -1.9 | 0.06 | . |  |
| factor(CPS_no) |  |  |  |  |  | 530 |
| 1 | -0.3 | 0.3 | -1.0 | 0.3 | ns |  |
| 2 | -0.6 | 0.3 | -2.0 | 0.04 | * |  |
| 3 | -1.6 | 0.5 | -3.2 | 0.0013 | ** |  |
| UC |  |  |  |  |  | 527 |
| TRUE | -0.8 | 0.5 | -1.7 | 0.09 | . |  |

**Example 3 - Prediction of aneuploid embryos**

As example 1 this analysis is based on known implantation data (KID) of 407 embryos incubated under different conditions (patient characteristics, clinical practices and rules and regulations). In this example 351 embryos are selected because they had annotations for tBI and tBE. The KID embryos are all transferred embryos with known implantation. With multiple embryo transfers only total failure of implantation or total success is used. All multiple transfers with implantation that have less implanted embryos than transferred were discarded to enable the implantation success for the specific embryo. Implantation successes (squares) and failures (triangles) have been plotted in fig. 16 with tEB along the first axis and tB along the second axis (where tBI is referred to as "tB").

The chart in fig. 16 have been divided into three sections "1", "2" and "3" by a vertical line at tEB = 120.3 hours and a horizontal line at tBI = 96.5 hours. Thus,

- In section "1" tEB is in the range of 80 to 120.3 hours and tBI is in the range of 70 to 96.5 hours.

- In section "2" tEB is in the range of 80 to 120.3 hours and tBI is in the range of 96.5 to 140 hours.

- In section "3" tEB is in the range of 120.3 to 150 hours and tBI is in the range of 70 to 140 hours.

The outcome of the embryos of these three sections is very different. The implantation ratio of embryos falling within section "1" is 0.59, the implantation ratio of embryos falling within section "2" is 0.37, whereas the implantation ratio of embryos falling within section "3" is 0.14. This is also visible in fig. 16 with an overweight of triangles in section "3" and an overweight of squares in section "1". These large observed differences in implantation ratio may be caused by different occurrences of aneuploidy, where the best implanting class in section "1" has a low occurrence of aneuploidy, the poorest implanting class in section "3" has a high degree of aneuploidy and the medium class in section "2" lies in between. Thus, tEB and tBI are very good candidates for blastocyst quality criteria. But tEB and tBI may also be used for distinguishing between euploid and aneuploid embryos and consequently by measuring morphological embryo parameters a non-invasive method for distinguishing between aneuploid and euploid embryos may be provided.

**References**

[1] Alikani M, Calderon G, Tomkin G et al. (2000) Cleavage anomalies in early human embryos and survival after prolonged culture in-vitro. Hum Reprod 15, 2634-2643.
[2] Wong CC, Loewke KE, Bossert NL et al. (2010) Non-invasive imaging of human embryos before embryonic genome activation predicts development to the blastocyst stage. Nat Biotechnol 28, 1115-1121.
[3] Kroener L et al. (2012) The effect of timing of embryonic progression on chromosomal abnormality. Fertility and Sterility Vol. 98, No. 4, 876-880.

**Further details of the invention**

The invention will now be described in further detail with reference to the following items:

1. A method for determining embryo quality comprising monitoring the embryo for a time period, and determining one or more quality criteria for said embryo, and based on said one or more quality criteria determining the embryo quality.

2. A method for determining embryo quality comprising monitoring the embryo for a time period, said time period comprising the transformation of the embryo from initial compaction or morula to blastocyst and determining one or more blastocyst quality criteria for said embryo, and based on said one or more blastocyst quality criteria determining the embryo quality.

3. The method according to any of the preceding items, wherein the embryo quality is determined from a plurality of said quality criteria and/or said blastocyst quality criteria, such as by combining a plurality of said quality criteria and/or said blastocyst quality criteria.

4. The method according to any of the preceding items, wherein the blastocyst quality criterion is a criterion relating to the phase from a 5 blastomere embryo to a blastocyst stage.

5. The method according to any of the preceding items, wherein the blastocyst quality criterion is a criterion relating to the phase wherein only the blastomeres own DNA is transcribed.

6. The method according to any of the preceding items, wherein a population of embryos is monitored.

7. The method according to any of the preceding items, wherein the embryo quality is a quality relating to implantation success.

8. The method according to any of the preceding items, wherein said one or more blastocyst quality criteria are combined with one or more exclusion criteria for deselecting and/or excluding embryos with a low probability of implantation success.

9. The method according to any of the preceding items, wherein the blastocyst stage is selected from the group of:

initial compaction (IC), morula (M), initial differentiation of trophectoderm cells (IDT), early blastocyst (ERB), blastocyst (BI), expansion of blastocyst (EB), first contraction (CPS(1)), second contraction (CPS(2)), third contraction (CPS(3)), fourth contraction (CPS(4)), fifth contraction (CPS(5)), sixth contraction (CPS(6)), seventh contraction (CPS(7)), hatching (HB), and fully hatched (FH).

10. The method according to any of the preceding items, wherein said time period comprises the time from fertilization to transformation to blastocyst and wherein fast development from fertilization to a 5-8 blastomere embryo relative to a slow development from the 5-8 blastomere embryo to a blastocyst stage is an indicator of high embryo quality.

11. The method according to any of preceding items, wherein said time period comprises the time from fertilization to a blastocyst stage and wherein fast development of the embryo when maternally inherited mRNA is translated relative to a slow development of the embryo when the blastomeres own DNA is transcribed is an indicator of high embryo quality.

12. The method according to any of preceding items, wherein said time period comprises the time from fertilization to a blastocyst stage wherein a quality criterion is determination of 1) the duration of a first time period wherein only maternally inherited mRNA is translated and 2) the duration of a second time period wherein only the blastomeres own DNA is transcribed.

13. The method according to any of preceding items, wherein said time period comprises the time from fertilization to a blastocyst stage wherein a quality criterion is determination of 1) the duration of a first time period from fertilization to a 5 blastomere embryo and 2) the duration of a second time period from the 5 blastomere embryo to said blastocyst stage.

14. The method according to any of preceding items, wherein said time period comprises the time from fertilization to a blastocyst stage wherein a quality criterion is determination of 1) the duration of a first time period from fertilization until translation of maternally inherited mRNA in the blastomeres is completed and 2) the duration of a second time period from initiation of transcription of the blastomeres own DNA to said blastocyst stage.

15. The method according to any of the preceding items 12 to 14, wherein a blastocyst quality criterion is the ratio of said first and second time periods.

16. The method according to item 15, wherein the ratio of the second time period divided by the first time period is an indicator of high embryo quality if said ratio is greater than a predefined value.

17. The method according to any of the preceding items 12 to 16, wherein a long duration of the first time period relative to a short duration of the second time period is an indicator of high embryo quality.

18. The method according to any of the preceding items 12 to 17, wherein said first period is defined as t5 and said second time period is defined as tEB.

19. The method according to item 18, wherein said first period being less than said second period is an indicator of high embryo quality.

20. The method according to any of the preceding items 18 to 19, wherein said blastocyst quality criterion is an indicator of high embryo quality if said ratio is greater than or equal to 1.08.

21. The method according to any of the preceding items 18 to 19, wherein said blastocyst quality criterion is an indicator of high embryo quality if said ratio is greater than 1, or greater than 1.01, or greater than 1.02, or greater than 1.02, or greater than 1.03, or greater than 1.04, or greater than 1.05, or greater than 1.06, or greater than 1.07, or greater than 1.08, or greater than 1.09, or greater than 1.10, or greater than 1.11, or greater than 1.12, or greater than 1.13, or greater than 1.14, or greater than 1.15, or greater than 1.16.

22. The method according to any of the preceding items, wherein said time period comprises the time from fertilization to transformation to blastocyst and wherein fast development from fertilization to a blastocyst stage combined with a slow development from the 5-8 blastomere embryo to a blastocyst stage is an indicator of high embryo quality.

23. The method according to any of the preceding items, wherein said time period comprises the time from fertilization to a blastocyst stage wherein a quality criterion is determination of 1) the duration of a first time period from fertilization to blastocyst, and 2) the duration of a second time period from initiation of transcription of the blastomeres own DNA to said blastocyst stage.

24. The method according to any of the preceding items 22 to 23, wherein a quality criterion is the ratio of said first and second time periods.

25. The method according to any of the preceding items, wherein a blastocyst quality criterion is determination of $tB_i - t_i$ wherein $tB_i$ is selected from the group of {tM, tBI, tEB} and $t_i$ is selected from the group of {t5, t6, t7 and t8}.

26. The method according to item 25, wherein said blastocyst quality criterion is an indicator of high embryo quality if tM - t8 is greater than or equal to 27.3 hours.

27. The method according to item 25, wherein said blastocyst quality criterion is an indicator of high embryo quality if tM - t8 is greater than 24 hours, or greater than 24.5 hours, or greater than 24.5 hours, or greater than 25 hours, or greater than 25.5 hours, or greater than 26 hours, or greater than 26.5 hours, or greater than 27 hours, or greater than 27.3 hours, or greater than 27.6 hours, or greater than 28 hours, or greater than 28.5 hours, or greater than 29 hours, or greater than 29.5 hours, or greater than 30 hours.

28. The method according to any of the preceding items, wherein a blastocyst quality criterion is determination of $(tB_i - t_i) / t_i$ wherein $tB_i$ is selected from the group of tM, tBI, tEB} and $t_i$ is selected from the group of {t5, t6, t7 and t8}.

29. The method according to item 28, wherein said blastocyst quality criterion is an indicator of high embryo quality if $(tB_i - t_i) / t_i$ is greater than a predefined value.

30. The method according to item 28, wherein said blastocyst quality criterion is an indicator of high embryo quality if (tEB - t5) / t5 is greater than or equal to 1.08.

31. The method according to any of the preceding items, wherein a blastocyst quality criterion is determination of $(tB_i - t_i) / tB_i$ wherein $tB_i$ is selected from the group of {tM, tBI, tEB} and $t_i$ is selected from the group of {t5, t6, t7 and t8}.

32. The method according to item 31, wherein said blastocyst quality criterion is an indicator of high embryo quality if $(tB_i - t_i) / tB_i$ is greater than a predefined value.

33. The method according to item 31, wherein said blastocyst quality criterion is an indicator of high embryo quality if (tEB - t5) / tEB is greater than or equal to 0.52.

34. The method according to item 31, wherein said blastocyst quality criterion is an indicator of high embryo quality if (tEB - t5) / tEB is greater than 0.45, or greater than 0.46, or greater than 0.47, or greater than 0.48, or greater than 0.49, or greater than 0.5, or greater than 0.51, or greater than 0.52, or greater than 0.53, or greater than 0.54, or greater than 0.55, or greater than 0.56, or greater than 0.57, or greater than 0.58, or greater than 0.59, or greater than 0.6.

35. The method according to any of the preceding items, wherein a blastocyst quality criterion is determination of the time from fertilization to a blastocyst stage.

36. The method according to any of the preceding items wherein a blastocyst quality criterion is determination of one or more of the following morphological blastocyst parameters:

tIC = time from fertilization to initial compaction,
tM = time from fertilization to Morula,
tIDT = time from fertilization to initial differentiation of trophectoderm cells,
tERB = time from fertilization to early blastocyst / onset of cavitation,
tBI = time from fertilization to blastocyst,
tEB = time from fertilization to expansion of blastocyst,
tCPS(1) = time from fertilization to first contraction,
tCPS(2) = time from fertilization to second contraction,
tCPS(3) = time from fertilization to third contraction,
tHB = time from fertilization to hatching, and
tFH = time from fertilization to fully hatched.

37. The method according to any of the preceding items, wherein a blastocyst quality criterion is determination of tIC and wherein said blastocyst quality criterion is an indicator of high embryo quality if tIC is between 72.4 and 79 hours.

38. The method according to any of the preceding items, wherein a blastocyst quality criterion is determination of tIC and wherein said blastocyst quality criterion is an indicator of high embryo quality if tIC is greater than 70 hours, or greater than 70.5 hours, or greater than 71 hours, or greater than 71.5 hours, or greater than 72 hours, or greater than 72.2 hours, or greater than 72.4 hours, or greater than 72.6 hours, or greater than 72.8 hours, or greater than 73 hours, or greater than 73.5 hours, or greater than 74 hours, or greater than 74.5 hours, or greater than 75 hours.

39. The method according to any of the preceding items, wherein a blastocyst quality criterion is determination of tIC and wherein said blastocyst quality criterion is an indicator of high embryo quality if tIC is less than 81 hours, or less than 80.5 hours, or less than 80 hours, or less than 79.5 hours, or less than 79 hours, or less than 78.5 hours, or less than 78 hours, or less than 77.5 hours, or less than 77 hours.

40. The method according to any of the preceding items, wherein a blastocyst quality criterion is determination of tM and wherein said blastocyst quality criterion is an indicator of high embryo quality if tM is less than 85.6 hours.

41. The method according to any of the preceding items, wherein a blastocyst quality criterion is determination of tM and wherein said blastocyst quality criterion is an indicator of high embryo quality if tM is less than 88 hours, or less than 87.5, or less than 87 hours, or less than 86.5 hours, or less than 86 hours, or less than 85.8 hours, or less than 85.6 hours, or less than 85.4 hours, or less than 85.2 hours, or less than 85 hours, or less than 84.5 hours, or less than 84 hours, or less than 83.5 hours, or less than 83 hours.

42. The method according to any of the preceding items, wherein a blastocyst quality criterion is determination of tIDT and wherein said blastocyst quality criterion is an indicator of high embryo quality if tIDT is less than 88.9 hours.

43. The method according to any of the preceding items, wherein a blastocyst quality criterion is determination of tIDT and wherein said blastocyst quality criterion is an indicator of high embryo quality if tIDT is less than 91 hours, or less than 90.5 hours, or less than 90 hours, or less than 89.5 hours, or less than 89 hours, or less than 88.9 hours, or less than 88.8 hours, or less than 88.5 hours, or less than 88 hours, or less than 87.5 hours, or less than 87 hours.

44. The method according to any of the preceding items, wherein a blastocyst quality criterion is determination of tBI and wherein said blastocyst quality criterion is an indicator of high embryo quality if tBI is less than 96.3 hours.

45. The method according to any of the preceding items, wherein a blastocyst quality criterion is determination of tBI and wherein said blastocyst quality criterion is an indicator of high embryo quality if tBI is less than 99 hours, or less than 98.5 hours, or less than 98 hours, or less than 97.5 hours, or less than 97 hours, or less than 96.7 hours, or less than 96.5 hours, or less than 96.3 hours, or less than 96 hours, or less than 95.5 hours, or less than 95 hours, or less than 94.5 hours, or less than 94 hours, or less than 93.5 hours, or less than 93 hours.

46. The method according to any of the preceding items, wherein a blastocyst quality criterion is determination of tEB and wherein said blastocyst quality criterion is an indicator of high embryo quality if tEB is less than 101.3 hours.

47. The method according to any of the preceding items, wherein a blastocyst quality criterion is determination of tEB and wherein said blastocyst quality criterion is an indicator of high embryo quality if tEB is less than 104 hours, or less than 103 hours, or less than 102.5 hours, or less than 102 hours, or less than 101.5 hours, or less than 101.3 hours, or less than 101 hours, or less than 100.5 hours, or less than 100 hours, or less than 99.5 hours, or less than 99 hours, or less than 98.5 hours, or less than 98 hours.

48. The method according to any of the preceding items, wherein a blastocyst quality criterion is determination of tEB and wherein said blastocyst quality criterion is an indicator of high embryo quality if tEB is less than 130 hours, or less than 129 hours, or less than 128 hours, or less than 127 hours, or less than 126 hours, or less than 125 hours, or less than 124.5 hours, or less than 124 hours, or less than 123.5 hours, or less than 123 hours, or less than 122.5 hours, or less than 122 hours, or less than 121.5 hours, or less than 121 hours, or less than 120.5 hours, or less than 120.3 hours, or less than 120.1 hours, or less than 120 hours, or less than 119.5 hours, or less than 119 hours, or less than 118.5 hours, or less than 118 hours, or less than 117.5 hours, or less than 117 hours, or less than 116.5 hours, or less than 116 hours, or less than 115.5 hours, or less than 115 hours, or less than 114.5 hours, or less than 114 hours, or less than 113.5 hours, or less than 113 hours, or less than 112.5 hours, or less than 112 hours, or less than 111.5 hours, or less than 111 hours, or less than 110.5 hours, or less than 110 hours, or less than 109.5 hours, or less than 109 hours, or less than 108.5 hours, or less than 108 hours, or less than 107.5 hours, or less than 107 hours, or less than 106.5 hours, or less than 106 hours, or less than 105.5 hours, or less than 105 hours.

49. The method according to any of the preceding items, wherein a blastocyst quality criterion is determination of tEB and tBI.

50. The method according to any of the preceding items, wherein a blastocyst quality criterion is determination of tEB and tBI and wherein said blastocyst quality criterion is an indicator of high embryo quality if tEB is less than 120.3 hours and tBI is less than 96.5 hours.

51. The method according to any of the preceding items, wherein a blastocyst quality criterion is determination of tEB and tBI and wherein said blastocyst quality criterion is an indicator of high embryo quality if tEB is less than130 hours, or less than 129 hours, or less than 128 hours, or less than 127 hours, or less than 126 hours, or less than 125 hours, or less than 124.5 hours, or less than 124 hours, or less than 123.5 hours, or less than 123 hours, or less than 122.5 hours, or less than 122 hours, or less than 121.5 hours, or less than 121 hours, or less than 120.5 hours, or less than 120.3 hours, or less than 120.1 hours, or less than 120 hours, or less than 119.5 hours, or less than 119 hours, or less than 118.5 hours, or less than 118 hours, or less than 117.5 hours, or less than 117 hours, or less than 116.5 hours, or less than 116 hours, or less than 115.5 hours, or less than 115 hours, or less than 114.5 hours, or less than 114 hours, or less than 113.5 hours, or less than 113 hours, or less than 112.5 hours, or less than 112 hours, or less than 111.5 hours, or less than 111 hours, or less than 110.5 hours, or less than 110 hours, or less than 109.5 hours, or less than 109 hours, or less than 108.5 hours, or less than 108 hours, or less than 107.5 hours, or less than 107 hours, or less than 106.5 hours, or less than 106 hours, or less than 105.5 hours, or less than 105 hours, or less than 104 hours, or less than 103 hours, or less than 102.5 hours, or less than 102 hours, or less than 101.5 hours, or less than 101.3 hours, or less than 101 hours, or less than 100.5 hours, or less than 100 hours, or less than 99.5 hours, or less than 99 hours, or less than 98.5 hours, or less than 98 hours, and tBI is less than 99 hours, or less than 98.5 hours, or less than 98 hours, or less than 97.5 hours, or less than 97 hours, or less than 96.7 hours, or less than 96.5 hours, or less than 96.3 hours, or less than 96 hours, or less than 95.5 hours, or less than 95 hours, or less than 94.5 hours, or less than 94 hours, or less than 93.5 hours, or less than 93 hours.

52. The method according to any of the preceding items, wherein a blastocyst quality criterion is determination of tCPS(1) and wherein said blastocyst quality criterion is an indicator of high embryo quality if tCPS(1) is less than 104 hours.

53. The method according to any of the preceding items, wherein a blastocyst quality criterion is determination of tCPS(1) and wherein said blastocyst quality criterion is an indicator of high embryo quality if tCPS(1) is less than 107 hours, or less than 106 hours, or less than 105.5 hours, or less than 105 hours, or less than 104.5 hours, or less than 104 hours, or less than 103.5 hours, or less than 103 hours, or less than 102.5 hours, or less than 102 hours, or less than 101 hours.

54. The method according to any of the preceding items, wherein a blastocyst quality criterion is determination of tHB and wherein said blastocyst quality criterion is an indicator of high embryo quality if tHB is less than 109.5 hours.

55. The method according to any of the preceding items, wherein a blastocyst quality criterion is determination of tHB and wherein said blastocyst quality criterion is an indicator of high embryo quality if tHB is less than 113 hours, or less than 112 hours, or less than 111.5 hours, or less than 111 hours, or less than 110.5 hours, or less than 110 hours, or less than 109.5 hours, or less than 109 hours, or less than 108.5 hours, or less than 108 hours, or less than 107.5 hours, or less than 107 hours, or less than 106.5 hours, or less than 106 hours.

56. The method according to any of the preceding items wherein a blastocyst quality criterion is determination of one or more of the following morphological blastocyst parameters:

tBI - tM,
tEB - tBI,
tHE - tEB,
tEB - tM
tBI - tCPS(1),
tBI - tCPS(2),
tEB - tCPS(1),
tEB - tCPS(2),
tCPS(2) - tCPS(1),
tCPS(3) - tCPS(2).

57. The method according to any of the preceding items, wherein a blastocyst quality criterion is determination of tBI - tCPS(2) and wherein said blastocyst quality criterion is an indicator of high embryo quality if tBI - tCPS(2) is greater than or equal to 10.4 hours.

58. The method according to any of the preceding items, wherein a blastocyst quality criterion is determination of tBI - tCPS(2) and wherein said blastocyst quality criterion is an indicator of high embryo quality if tBI - tCPS(2) is greater than 8 hours, or greater than 8.5 hours, or greater than 9 hours, or greater than 9.5 hours, or greater than 10 hours, or greater than 10.2 hours, or greater than 10.4 hours, or greater than 10.6 hours, or greater than 10.8 hours, or greater than 11 hours, or greater than 11.5 hours, or greater than 12 hours, or greater than 12.5 hours, or greater than 13 hours.

59. The method according to any of the preceding items, wherein a blastocyst quality criterion is determination of tEB - tCPS(1) and wherein said blastocyst quality criterion is an indicator of high embryo quality tEB - tCPS(1) is greater than -3.3 hours and less than 2.2 hours.

60. The method according to any of the preceding items, wherein a blastocyst quality criterion is determination of tEB - tCPS(1) and wherein said blastocyst quality criterion is an indicator of high embryo quality tEB - tCPS(1) is greater than -5 hours, or greater than -4.5 hours, or greater than -4 hours, or greater than -3.5 hours, or greater than -3.3 hours, or greater than -3.1 hours, or greater than -3 hours, or greater than -2.5 hours, or greater than -2 hours, or greater than -1.5 hours, or greater than -1 hours.

61. The method according to any of the preceding items, wherein a blastocyst quality criterion is determination of tEB - tCPS(1) and wherein said blastocyst quality criterion is an indicator of high embryo quality tEB - tCPS(1) is less than 4 hours, or less than 3.5 hours, or less than 3 hours, or less than 2.8 hours, or less than 2.5 hours, or less than 2.2 hours, or less than 2 hours, or less than 1.5 hours, or less than 1 hour.

62. The method according to any of the preceding items, wherein a blastocyst quality criterion is determination of tCPS(2) - tCPS(1) and wherein said blastocyst quality criterion is an indicator of high embryo quality tCPS(2) - tCPS(1) is greater than or equal to 7.76 hours.

63. The method according to any of the preceding items, wherein a blastocyst quality criterion is determination of tCPS(2) - tCPS(1) and wherein said blastocyst quality criterion is an indicator of high embryo quality tCPS(2) - tCPS(1) is greater than 5 hours, or greater than 6 hours, or greater than 6.5 hours, or greater than 7 hours, or greater than 7.3 hours, or greater than 7.6 hours, or greater than 7.8 hours, or greater than 8 hours, or greater than 8.2 hours, or greater than 8.5 hours, or greater than 9 hours, or greater than 9.5 hours, or greater than 10 hours.

64. The method according to any of the preceding items, wherein the blastocyst quality criterion is selected from the group of normalized morphological blastocyst parameters.

65. The method according to any of the preceding items, wherein the quality criterion is selected from the group of normalized morphological blastocyst parameters relating to the phase from the 5 or 8 blastomere embryo to transformation into blastocyst.

66. The method according to any of the preceding items, wherein a quality criterion is based on the ratio of two time intervals, each of said time intervals is determined as the duration of a time period between two morphological and/or kinetic events in the embryo development.

67. The method according to item 66, wherein said quality criterion is a normalized morphological embryo parameter.

68. The method according to any of the preceding items, wherein a blastocyst quality criterion is determination of the absolute or relative 2D and/or 3D expansion of the blastocyst.

69. The method according to any of the preceding items, wherein a blastocyst quality criterion is determination of the diameter and/or the volume of the embryo at the onset of expansion.

70. The method according to any of the preceding items, wherein a blastocyst quality criterion is determination of the maximum diameter and/or the maximum volume of the blastocyst before hatching.

71. The method according to any of the preceding items, wherein an exclusion criterion is determination of the number of contractions CPS_no of the blastocyst and wherein a CPS_no of more than 2 is an indicator of low embryo quality.

72. The method according to any of the preceding items, wherein an exclusion criterion is determination of the degree of vacuolization VC_grade of the blastocyst and wherein a VC_grade of more than 1 is an indicator of low embryo quality.

73. The method according to any of the preceding items, wherein an exclusion criterion is determination of uneven compaction and wherein an uneven compaction is an indicator of low embryo quality.

74. The method according to any of preceding items, wherein the time for cleavage to a 5 blastomere embryo is determined.

75. The method according to any of preceding items, wherein the time for cleavage to an 8 blastomere embryo is determined.

76. The method according to any of preceding items, wherein the time for cleavage to a 2 blastomere embryo, a 3 blastomere embryo, a 4 blastomere embryo, a 5 blastomere embryo, a 6 blastomere embryo, a 7 blastomere embryo, and/or an 8 blastomere embryo.

77. The method according to any of the preceding items, wherein a quality criterion is determination of the time for cleavage to a 2 blastomere embryo, a 3 blastomere embryo, a 4 blastomere embryo, a 5 blastomere embryo, a 6 blastomere embryo, a 7 blastomere embryo, and/or an 8 blastomere embryo.

78. The method according to any of the preceding items, wherein the quality criterion is selected from the group of normalized morphological embryo parameters.

79. The method according to any of the preceding items, wherein the quality criterion is selected from the group of normalized morphological embryo parameters relating to the phase of from 2 to 8 blastomere embryo.

80. The method according to any of the preceding items, wherein a quality criterion is a normalized morphological embryo parameter based on two, three, four, five or more parameters selected from the group of t2, t3, t4, t5, t6, t7, t8 and t9.

81. The method according to any of the preceding items, wherein a quality criterion is a normalized morphological embryo parameter based on four parameters selected from the group of t2, t3, t4, t5, t6, t7, t8 and t9.

82. The method according to any of the preceding items, wherein a quality criterion is based on the ratio of two time intervals, each of said time intervals determined as the duration of a time period between two morphological events in the embryo development.

83. The method according to item 66, wherein said quality criterion is a normalized morphological embryo parameter.

84. The method according to any of the preceding items 66 to 67, wherein said morphological events are selected from the group of fertilization, initiation of a blastomere cleavage and completion of a blastomere cleavage.

85. The method according to any of the preceding items, wherein a quality criterion is determination of

$$CC_{norm} = \sqrt{\sum_{all\ i} \left( \frac{CCi - CCi_{median}}{CCi_{median}} \right)^2},$$ where CCi is the duration of a cell cycle.

86. The method according to item 85, wherein a low value of $CC_{norm}$ indicates a high embryo quality and/or a high value of $CC_{norm}$ indicates a poor embryo quality.

87. The method according to any of the preceding items, wherein a quality criterion is determination of

$$S_{norm} = \sqrt{\sum_{all\ i} \left( \frac{Si}{Si_{median}} \right)^2}$$ where Si is the synchrony of a division.

88. The method according to item 87, wherein a low value of $S_{norm}$ indicates a high embryo quality and/or a high value of $S_{norm}$ indicates a poor embryo quality.

89. The method according to any of the preceding items 56 to 84, wherein the normalized morphological embryo parameter is selected from the group of

cc2/cc2_3 = (t3-t2)/(t5-t2),

cc3/cc2_3 = (t5-t3)/(t5-t2),

cc3/t5 = 1 - t3/t5,

s2/cc2 = (t4-t3)/(t3-t2),

s3/cc3 = (t8-t5)/(t5-t3),

and

cc2/cc3 = (t3-t2)/(t5-t3).

90. The method according to any of the preceding items, wherein a quality criterion is determination of cc2/cc2_3 = (t3-t2)/(t5-t2).

91. The method according to item 90, wherein said quality criterion is an indicator of high embryo quality if cc2/cc2_3 = (t3-t2)/(t5-t2) is between 0.38 and 0.5, or between 0.39 and 0.49, or between 0.4 and 0.48 or between 0.41 and 0.47.

92. The method according to any of the preceding items, wherein the quality criterion is determination of t3/t5.

93. The method according to item 92, wherein said quality criterion is an indicator of high embryo quality if t3/t5 is greater than 0.6, or greater than 0.62, or greater than 0.64, or greater than 0.66, or greater than 0.68, or greater than 0.7, or greater than 0.72, or greater than 0.74.

94. The method according to any of the preceding items, wherein a quality criterion is determination of s2/cc2 = (t4-t3)/(t3-t2).

95. The method according to item 94, wherein said quality criterion is an indicator of high embryo quality if s2/cc2 = (t4-t3)/(t3-t2) is less than 0.03, or less than 0.029, or less than 0.028, or less than 0.027, or less than 0.026, or less than 0.025, or less than 0.024, or less than 0.023, or less than 0.022, or less than 0.021, or less than 0.02.

96. The method according to any of the preceding items, wherein a quality criterion is determination of s3/cc3 = (t8-t5)/(t5-t3).

97. The method according to item 94, wherein said quality criterion is an indicator of high embryo quality if s3/cc3 = (t8-t5)/(t5-t3) is less than 0.25, or less than 0.23, or less than 0.21, or less than 0.2, or less than 0.19, or less than 0.18, or less than 0.17, or less than 0.16, or less than 0.15.

98. The method according to any of the preceding items, wherein a quality criterion is determination of cc2/cc3 = (t3-t2)/(t5-t3).

99. The method according to item 98, wherein said quality criterion is an indicator of high embryo quality if cc2/cc3 = (t3-t2)/(t5-t3) is between 0.7 and 0.9, or between 0.71 and 0.89, or between 0.72 and 0.88.

100. The method according to any of the preceding items, wherein a quality criterion is determination of the extent of irregularity of the timing of cell divisions when the embryo develops from 4 to 8 blastomeres.

101. The method according to any of the preceding items, wherein a quality criterion is determination of the maximum cleavage time for each blastomere
when the embryo develops from 4 to 8 blastomeres.

102. The method according to item 101, wherein said quality criterion is an indicator of high embryo quality if said maximum cleavage time is less than 1.5 hours.

103. The method according to item 101, wherein said quality criterion is an indicator of high embryo quality if said maximum cleavage time is less than 2.5 hours, or less than 2.3 hours, or less than 2.1 hours, or less than 2 hours, or less than 1.9 hours, or less than 1.8 hours, or less than 1.7 hours, or less than 1.65 hours, or less than 1.6 hours, or less than 1.55 hours, or less than 1.5 hours, or less than 1.45 hours, or less than 1.4 hours, or less than 1.35 hours, or less than 1.3 hours, or less than 1.25 hours, or less than 1.2 hours, or less than 1.15 hours, or less than 1.1 hours, or less than 1 hour.

104. The method according to any of the preceding items, wherein a quality criterion is determination of the ratio between the maximum cleavage time for each blastomere when the embryo develops from 4 to 8 blastomeres and the duration of the total time period from 4 to 8 blastomeres; max(s3a,s3b,s3c)/s3.

105. The method according to item 104, wherein said quality criterion is a normalized morphological embryo parameter.

106. The method according to any of the preceding items 104 to 105, wherein said quality criterion is an indicator of high embryo quality if said ratio is less than 0.5.

107. The method according to any of the preceding items 104 to 105, wherein said quality criterion is an indicator of high embryo quality if said ratio is less than 0.8, or less than 0.75, or less than 0.7, or less than 0.65, or less than 0.6, or less than 0.58, or less than 0.56, or less than 0.54, or less than 0.52, or less than 0.5, or less than 0.48, or less than 0.46, or less than 0.44, or less than 0.42, or less than 0.4.

108. The method according to any of the preceding items, wherein a quality criterion is determination of the time for cleavage to a 5 blastomere embryo.

109. The method according to item 108, wherein said quality criterion is an indicator of high embryo quality if t5 is less than 58 hours, or less than 57 hours or less than 56.5 hours, or less than 56.3 hours, or less than 56.2 hours, or less than 56.1 hours, or less than 56 hours, or less than 55.9 hours, or less than 55.8 hours, or less than 55.7 hours, or less than 55.6 hours, or less than 55.5 hours, or less than 55 hours, or less than 54.5 hours

110. The method according to any of the preceding items 108 to 109, wherein said quality criterion is an indicator of high embryo quality if t5 is greater than 46 hours, or greater than 47 hours, or greater than 47 hours, or greater than 48 hours, or greater than 48.5 hours, or greater than 48.7 hours, or greater than 48.9 hours, or greater than 49 hours, or greater than 49.1 hours, or greater than 49.2 hours, or greater than 49.3 hours, or greater than 49.4 hours, or greater than 49.5 hours, or greater than 49.6 hours, or greater than 49.7 hours, or greater than 49.8 hours, or greater than 49.9 hours, or greater than 50 hours, or greater than 51 hours, or greater than 52 hours, or greater than 53 hours.

111. The method according to item 108, wherein said quality criterion is an indicator of high embryo quality ratio if t5 is between 48.7 and 55.6 hours.

112. The method according to any of the preceding items, wherein a quality criterion is determination of the time for cleavage to an 8 blastomere embryo, t8.

113. The method according to item 108, wherein said quality criterion is an indicator of high embryo quality if t8 is less than 60 hours, or less than 59 hours or less than 58 hours, or less than 57.8 hours, or less than 57.6 hours, or less than 57.4 hours, or less than 57.2 hours, or less than 57 hours, or less than 56.8 hours, or less than 56.6 hours, or less than 56.4 hours, or less than 56.2 hours, or less than 56 hours, or less than 55 hours.

114. The method according to any of the preceding items, wherein a quality criterion is determination of the second cell cycle length cc2.

115. The method according to item 114, wherein said quality criterion is an indicator of high embryo quality if cc2 = t3-t2 is less than 14 hours, or less than 13.5 hours, or less than 13 hours, or less than 12.9 hours, or less than 12.8 hours, or less than 12.7 hours, or less than 12.6 hours, or less than 12.5 hours, or less than 12.4 hours, or less than 12.3 hours, or less than 12.1 hours, or less than 12 hours, or less than 11.9 hours, or less than 11.9 hours, or less than 11.8 hours, or less than 11.7 hours, or less than 11.6 hours, or less than 11.5 hours, or less than 11.4 hours, or less than 11.3 hours, or less than 11.2 hours, or less than 11.1 hours, or less than 11 hours, or less than 10.9 hours, or less than 10.8 hours, or less than 10.7 hours, or less than 10.6 hours, or less than 10.5 hours, or less than 10 hours.

116. The method according to any of the preceding items, wherein a quality criterion is determination of cc2b = t4-t2.

117. The method according to item 116, wherein said quality criterion is an indicator of high embryo quality if cc2b = t4-t2 is less than 14 hours, or less than 13.9 hours, or less than 13.8 hours, or less than 13.7 hours, or less than 13.6 hours, or less than 13.5 hours, or less than 13.4 hours, or less than 13.3 hours, or less than 13.2 hours, or less than 13.1 hours, or less than 13 hours, or less than 12.9 hours, or less than 12.8 hours, or less than 12.7 hours, or less than 12.6 hours, or less than 12.5 hours, or less than 12.4 hours, or less than 12.3 hours, or less than 12.1 hours, or less than 12 hours, or less than 11.9 hours, or less than 11.9 hours, or less than 11.8 hours, or less than 11.7 hours, or less than 11.6 hours, or less than 11.5 hours, or less than 11.4 hours, or less than 11.3 hours, or less than 11.2 hours, or less than 11.1 hours, or less than 11 hours, or less than 10.9 hours, or less than 10.8 hours, or less than 10.7 hours, or less than 10.6 hours, or less than 10.5 hours, or less than 10 hours.

118. The method according to any of the preceding items, wherein a quality criterion is determination of the third cell cycle length cc3.

119. The method according to item 118, wherein said quality criterion is an indicator of high embryo quality if cc3 = t5-t3 is less than 19 hours, or less than 18.5 hours, or less than 18 hours, or less than 17.9 hours, or less than 17.8 hours, or less than 17.7 hours, or less than 17.6 hours, or less than 17.5 hours, or less than 17.4 hours, or less than 17.3 hours, or less than 17.2 hours, or less than 17.1 hours, or less than 17 hours, or less than 16.9 hours, or less than 16.8 hours, or less than 16.7 hours, or less than 16.6 hours, or less than 16.5 hours, or less than 16.4 hours, or less than 16.3 hours, or less than 16.2 hours, or less than 16.1 hours, or less than 16 hours, or less than 15.8 hours, or less than 15.6 hours, or less than 15.5 hours, or less than 15.4 hours, or less than 15.3 hours, or less than 15.1 hours, or less than 15 hours, or less than 14.9 hours, or less than 14.9 hours, or less than 14.8 hours, or less than 14.7 hours, or less than 14.6 hours, or less than 14.5 hours, or less than 14.4 hours, or less than 14.3 hours, or less than 14.2 hours, or less than 14.1 hours, or less than 14 hours, or less than 13 hours.

120. The method according to any of items 118 to 119, wherein said quality criterion is an indicator of high embryo quality if cc3 = t5-t3 is greater than 11 hours, or greater than 11.5 hours, or greater than 12 hours, or greater than 12.2 hours, or greater than 12.4 hours, or greater than 12.5 hours, or greater than 12.6 hours, or greater than 12.7 hours, or greater than 12.8 hours, or greater than 12.9 hours, or greater than 13 hours, or greater than 13.1 hours, or greater than 13.2 hours, or greater than 13.3 hours, or greater than 13.5 hours, or greater than 14 hours.

121. The method according to any of the preceding items, wherein a quality criterion is determination of cc2_3 = t5-t2.

122. The method according to item 121, wherein said quality criterion is an indicator of high embryo quality if cc2_3 = t5-t2 is less than 32 hours, or less than 31 hours, or less than 30 hours, or less than 29.8 hours, or less than 29.6 hours, or less than 29.5 hours, or less than 29.4 hours, or less than 29.3 hours, or less than 29.2 hours, or less than 29.1 hours, or less than 29 hours, or less than 28.9 hours, or less than 28.8 hours, or less than 28.7 hours, or less than 28.6 hours, or less than 28.5 hours, or less than 28.4 hours, or less than 28.2 hours, or less than 28 hours, or less than 27.5 hours, or less than 27 hours, or less than 26 hours.

123. The method according to any of the preceding items, wherein a quality criterion is determination of the synchrony in division from a 2 blastomere embryo to a 4 blastomere embryo s2 = t4-t3.

124. The method according to item 123, wherein said quality criterion is an indicator of high embryo quality if s2 = t4-t3 is less than 3 hours, or less than 2.8 hours, or less than 2.6 hours, or less than 2.4 hours, or less than 2.3 hours, or less than 2.2 hours, or less than 2.1 hours, or less than 2 hours, or less than 1.8 hours, or less than 1.6 hours, or less than 1.4 hours, or less than 1.2 hours, or less than 1 hour, or less than 0.9 hours, or less than 0.8 hours, or less than 0.7 hours, or less than 0.6 hours, or less than 0.5 hours, or less than 0.45 hours, or less than 0.4 hours, or less than 0.39 hours, or less than 0.38 hours, or less than 0.37 hours, or less than 0.36 hours, or less than 0.35 hours, or less than 0.34 hours, or less than 0.33 hours, or less than 0.32 hours, or less than 0.31 hours, or less than 0.3 hours, or less than 0.29 hours, or less than 0.28 hours, or less than 0.27 hours, or less than 0.26 hours, or less than 0.25 hours, or less than 0.24 hours, or less than 0.22 hours, or less than 0.2 hours.

125. The method according to any of the preceding items, wherein a quality criterion is determination of the synchrony in division from a 4 blastomere embryo to a 8 blastomere embryo s3 = t8-t5.

126. The method according to item 125, wherein said quality criterion is an indicator of high embryo quality if s3 = t8-t3 is less than 5 hours, or less than 4.5 hours, or less than 4.3 hours, or less than 4.2 hours, or less than 4.1 hours, or less than 4 hours, or less than 3.9 hours, or less than 3.8 hours, or less than 3.7 hours, or less than 3.6 hours, or less than 3.5 hours, or less than 3.4 hours, or less than 3.3 hours, or less than 3.2 hours, or less than 3.1 hours, or less than 3 hours, or less than 2.9 hours, or less than 2.8 hours, or less than 2.7 hours, or less than 2.6 hours, or less than 2.55 hours, or less than 2.53 hours, or less than 2.51 hours, or less than 2.5 hours, or less than 2.4 hours, or less than 2.3 hours, or less than 2.2 hours, or less than 2.1 hours, or less than 2 hours, or less than 1.8 hours, or less than 1.6 hours, or less than 1.4 hours, or less than 1.2 hours, or less than 1 hour.

127. The method according to any of the preceding items, wherein the quality criterion is combined with determination of second cell cycle length.

128. The method according to any of the preceding items, wherein the quality criterion is combined with determination of synchrony in cleavage from a 2 blastomere embryo to a 4 blastomere embryo.

129. The method according to any of the preceding items, wherein the quality criterion is a combination of determination of time for cleavage to a 5 blastomere embryo and determination of the second cell cycle length.

130. The method according to item 9, where the quality criterion is further combined with determination of synchrony in cleavage from 2 blastomere embryo to 4 blastomere embryo.

131. The method according to any of the preceding items, wherein the determination of embryo quality further includes i) determining the extent and/or spatial distribution of cellular or organelle movement during the cell cleavage period; and/or ii) determining the extent and/or spatial distribution of cellular or organelle movement during the inter-cleavage period thereby obtaining an embryo quality measure.

132. The method according to any of the preceding items, wherein the embryo is monitored for a time period comprising at least three cell cycles, such as at least four cell cycles.

133. The method according to any of the preceding items, wherein the length of each cleavage period is determined.

134. The method according to any of the preceding items, wherein the length of each inter-cleavage period is determined.

135. The method according to any of the preceding items, wherein the period of cellular movement in at least two inter-cleavage periods is determined.

136. The method according to any of the preceding items, wherein the extent of cellular movement is determined in at least two inter-cleavage periods.

137. The method according to any of the preceding items, wherein the quality measure includes at least one exclusion criterion.

138. The method according to any of preceding items, wherein the exclusion criterion includes information of blastomere evenness at t2, information of multi nuclearity at the two-blastomere stage and/or at the four-blastomere stage, and/or information of cleavage from one blastomere directly to three blastomeres.

139. The method according to any of the preceding items, wherein an exclusion criterion is that cc2 and/or cc3 is less than 10 hours, or less than 9.5 hours, or less than 9 hours, or less than 8.5 hours, or less than 8 hours, or less than 7.5 hours, or less than 7 hours, or less than 6.5 hours, or less than 6 hours, or less than 5.5 hours, or less than 5 hours, or less than 4.5 hours, or less than 4 hours, or less than 3.5 hours, or less than 3 hours, or less than 2.5 hours, or less than 2 hours, or less than 1.5 hours, or less than 1 hour.

140. The method according to any of the preceding items, wherein an exclusion criterion is that t2 is greater than 28 hours, or greater than 28.5 hours, or greater than 29 hours, or greater than 29.5 hours, or greater than 30 hours, or greater than 30.5 hours, or greater than 31 hours, or greater than 31.25 hours, or greater than 31.5 hours, or greater than 31.75 hours, or greater than 32 hours, or greater than 32.5 hours, or greater than 33 hours, or greater than 33.5 hours, or greater than 34 hours.

141. The method according to any of the preceding items, wherein an exclusion criterion is that cc2b is greater than 11 hours, or greater than 11.5 hours, or greater than 12 hours, or greater than 12.5 hours, or greater than 12.75 hours, or greater than 13 hours, or greater than 13.1 hours, or greater than 13.25 hours, or greater than 13.5 hours, or greater than 14 hours, or greater than 14.5 hours, or greater than 15 hours.

142. The method according to any of the preceding items, wherein an exclusion criterion is that cc3 is greater than 15 hours, or greater than 15.5 hours, or greater than 16 hours, or greater than 16.5 hours, or greater than 17 hours, or greater than 17.25 hours, or greater than 17.5 hours, or greater than 17.6 hours, or greater than 17.75 hours, or greater than 18 hours, or greater than 18.5 hours, or greater than 19 hours, or greater than 19.5 hours.

143. The method according to any of the preceding items, wherein an exclusion criterion is that s2 is greater than 1 hour, or greater than 1.1 hours, or greater than 1.2 hours, or greater than 1.3 hours, or greater than 1.4 hours, or greater than 1.5 hours, or greater than 1.6 hours, or greater than 1.7 hours, or greater than 1.8 hours, or greater than 1.9 hours, or greater than 2 hours, or greater than 2.1 hours, or greater than 2.2 hours, or greater than 2.3 hours, or greater than 2.4 hours, or greater than 2.5 hours, or greater than 2.6 hours, or greater than 2.7 hours, or greater than 2.8 hours, or greater than 2.9 hours, or greater than 3 hours.

144. The method according to any of the preceding items, wherein an exclusion criterion is that s3 is greater than 2 hours, or greater than 2.2 hours, or greater than 2.4 hours, or greater than 2.6 hours, or greater than 2.8 hours, or greater than 3 hours, than 3.1 hours, or greater than 3.2 hours, or greater than 3.3 hours, or greater than 3.4 hours, or greater than 3.5 hours, or greater than 3.6 hours, or greater than 3.7 hours, or greater than 3.8 hours, or greater than 3.9 hours, or greater than 4 hours, than 4.1 hours, or greater than 4.2 hours, or greater than 4.3 hours, or greater than 4.4 hours, or greater than 4.5 hours, or greater than 4.6 hours, or greater than 4.7 hours, or greater than 4.8 hours, or greater than 4.9 hours, or greater than 5 hours, or greater than 5.25 hours, or greater than 5.5 hours, or greater than 6 hours.

145. A method for detecting aneuploidy in an embryo comprising:

- monitoring the embryo for a time period, said time period at least comprising the transformation of the embryo from initial compaction or morula to a blastocyst stage,
- measuring one or more morphological embryo parameters of said embryo, and
- based on said one or more morphological embryo parameters determining if said human embryo is aneuploid.

146. The method according to item 145, wherein said time period comprises the time from fertilization to said blastocyst stage.

147. The method according to any of items 145 to 146, wherein said one or more morphological embryo parameters is one or more of the quality criteria of any of the preceding items 4 to 144, such as one or more of the blastocyst quality criteria of any of the preceding items 4 to 144.

148. The method according to any of items 145 to 147, wherein said one or more morphological embryo parameters is selected from the group of

tIC = time from fertilization to initial compaction,
tM = time from fertilization to morula,
tIDT = time from fertilization to initial differentiation of trophectoderm cells,
tERB = time from fertilization to early blastocyst,
tBI = time from fertilization to blastocyst,
tEB = time from fertilization to expansion of blastocyst,
tCPS(1) = time from fertilization to first contraction,
tCPS(2) = time from fertilization to second contraction,
tCPS(3) = time from fertilization to third contraction,
tHB = time from fertilization to hatching, and
tFH = time from fertilization to fully hatched.

149. The method according to item 148, wherein aneuploidy is detected if one or more of said parameters falls outside their normal range.

150. The method according to any of items 145 to 149 wherein said morphological embryo parameters are

tBI = time from fertilization to blastocyst, and
tEB = time from fertilization to expansion of blastocyst.

151. The method according to any of items 145 to 150, wherein aneuploidy is detected if both tBI and tEB fall outside their normal range.

152. The method of item 151, wherein the normal range of tBI is less than 96.5 hours and the normal range of tEB is less than 120.3 hours.

153. The method of item 151, wherein the normal range of tBI is between 75 and 96.5 hours and the normal range of tEB is between 80 and 120.3 hours.

154. The method according to any of items 1 to 144, wherein aneuploidy detected according to the method of any of items 145 to 153 is an exclusion criterion.

155. The method according to any of the preceding items, wherein the embryo is monitored in an incubator.

156. The method according to any of preceding items, wherein the embryos are monitored by means of time-lapse microscopy equipment.

157. The method according to any of preceding items, wherein the morphological embryo and blastocyst parameters are determined by analysing time-lapse image series acquired by means of time-lapse microscopy equipment.

158. The method according to any of preceding items, wherein the embryos are monitored during cultivation of said embryos which are positioned in a culture medium.

159. The method according to any of preceding items, wherein the embryos are human embryos.

160. The method according to any of preceding items, further comprising the step of selecting the embryo having the highest embryo quality measure and transplanting said embryo to a recipient.

161. A system for determining embryo quality comprising means for monitoring the embryo for a time period, said system further having means for determining a quality criteria for said embryo, and having means for determining the embryo quality based on said quality criteria.

162. The system according to item 161, comprising means for determining one or more of the features as defined in any of the items 1 - 160.


**Claims**

1. A method for determining human embryo quality comprising monitoring the embryo for a time period from fertilization to the expansion of the blastocyst using time-lapse microscopy equipment, determining the blastocyst quality criteria tEB and tBI, where tEB indicates a time from fertilisation to expansion of blastocyst and tBI indicates a time from fertilisation to blastocyst, for said embryo, and based on tEB and tBI determining the embryo quality, wherein said blastocyst quality criteria is an indicator of high embryo quality if tEB is less than 122.5 hours and tBI is less than 96.3 hours.

2. The method of claim 1, further comprising monitoring the time from fertilization to a blastocyst stage, and wherein 1) the duration of a first time period from fertilization until translation of maternally inherited mRNA in the blastomeres is completed and 2) the duration of a second time period from initiation of transcription of the blastomeres own DNA to said blastocyst stage are determined, and wherein a blastocyst quality criterion is the ratio of said first and second time periods, and based on said one or more blastocyst quality criteria determining the embryo quality.

3. The method according to claim 2, wherein said blastocyst stage is selected from the group of: initial compaction (IC), morula (M), initial differentiation of trophectoderm cells (IDT), early blastocyst (ERB), blastocyst (BI), expansion of blastocyst (EB), first contraction (CPS(1)), second contraction (CPS(2)), third contraction (CPS(3)), fourth contraction (CPS(4)), fifth contraction (CPS(5)), sixth contraction (CPS(6)), seventh contraction (CPS(7)), hatching (HB), and fully hatched (FH).

4. The method according to claim 2, wherein said first period is defined as t4 and said second time period is defined as tEB -t8 or wherein said first period is defined as t5 and said second time period is defined as tEB -t5.

5. The method according to any of the preceding claims 2 to 4, wherein the ratio defined as the second time period divided by the first time period is an indicator of high embryo quality if said ratio is greater than a predefined value.

6. The method according to any of the preceding claims 2 to 5, wherein a long duration of the first time period relative to a short duration of the second time period is an indicator of high embryo quality.

7. The method according to any of the preceding claims 5 to 6, wherein said blastocyst quality criterion is an indicator of high embryo quality if said ratio is greater than or equal to 1.08.

8. The method according to any of the preceding claims 2 to 7, wherein a blastocyst quality criterion is determination of tM - t8, and wherein said blastocyst quality criterion is an indicator of high embryo quality if tM - t8 is greater than or equal to 27.3 hours.

9. The method according to any of the preceding claims 2 to 8, wherein a blastocyst quality criterion is determination of tIC and wherein said blastocyst quality criterion is an indicator of high embryo quality if tIC is between 72.4 and 79 hours.

10. The method according to any of the preceding claims1 to 9, wherein a blastocyst quality criterion is determination of tBI - tCPS(2) and wherein said blastocyst quality criterion is an indicator of high embryo quality if tBI - tCPS(2) is greater than or equal to 10.4 hours.

11. The method according to any of the preceding claims 1 to 10, wherein a blastocyst quality criterion is determination of tCPS(2) - tCPS(1) and wherein said blastocyst quality criterion is an indicator of high embryo quality if tCPS(2) - tCPS(1) is greater than or equal to 7.76 hours.

12. The method according to any of the preceding claims 2 to 11, wherein a blastocyst quality criterion is determination of the absolute or relative 2D and/or 3D expansion of the blastocyst.

13. The method according to any of the preceding claims 2 to 12, wherein the diameter and/or the volume of the embryo at the onset of expansion is determined and wherein the maximum diameter and/or the maximum volume of the blastocyst before hatching is determined and wherein a blastocyst quality criterion is the ratio of said diameters and/or wherein a blastocyst quality criterion is the ratio of said volumes.

14. The method according to claim 1, wherein a blastocyst quality criterion is a determination of: tCPS(1), and/or tBI - tCPS(2), and/or tEB - tCPS(1), and/or tCPS(2) - tCPS(1), and wherein said blastocyst quality criterion is an indicator of high embryo quality if: tCPS(1) is less than 107 hours, tBI-tCPS(2) is greater than 8 hours, tEB - tCPS(1) is less than 4 hours or greater than -5 hours, or tCPS(2) - tCPS(1) is greater than 5 hours, wherein tCPS(1) is the time from fertilization to first contraction and tCPS(2) is the time from fertilization to second contraction.

## Patentansprüche

1. Verfahren zum Bestimmen der Qualität eines menschlichen Embryos, das das Überwachen des Embryos über einen Zeitabschnitt von der Fertilisierung bis zur Expansion des Blastozysten umfasst, wobei Videomikroskopie-Ausrüstung verwendet wird, Bestimmen der Blastozysten-Qualitätskriterien tEB und tBI, wobei tEB eine Zeit von der Fertilisierung bis zur Expansion des Blastozysten angibt und tBI eine Zeit von der Fertilisierung bis zum Blastozysten für den Embryo angibt, und auf der Basis von tEB und tBI, Bestimmen der Qualität des Embryos, wobei die Qualitätskriterien des Blastozysten ein Indikator für hohe Embryoqualität sind, wenn tEB kleiner als 122,5 Stunden ist und tBI kleiner als 96,3 Stunden ist.

2. Verfahren nach Anspruch 1, das ferner das Überwachen der Zeit von der Fertilisierung bis zu einem Blastozystenstadium umfasst, und wobei 1) die Dauer einer ersten Zeitperiode von der Fertilisierung bis zur Translation von mütterlich geerbter mRNA in die Blastomeren abgeschlossen ist und 2) die Dauer einer zweiten Zeitperiode vom Beginn der Transkription der eigenen DNA der Blastomeren bis zum Blastozystenstadium bestimmt werden, und wobei ein Blastozystenqualitätskriterium das Verhältnis der ersten zur zweiten Zeitperiode ist, und auf der Basis des einen oder der mehreren Blastozystenqualitätskriterien, Bestimmen der Embryoqualität.

3. Verfahren nach Anspruch 2, wobei das Blastozystenstadium ausgewählt wird aus der Gruppe, bestehend aus: anfängliche Verdichtung (IC), Morula (M), anfängliche Differenzierung von Trophektodermzellen (IDT), früher Blastozyst (ERB), Blastozyst (BI), Expansion des Blastozysten (EB), erste Kontraktion (CPS(1)), zweite Kontraktion (CPS(2)), dritte Kontraktion (CPS(3)), vierte Kontraktion (CPS(4)), fünfte Kontraktion (CPS(5)), sechste Kontraktion (CPS(6)), siebente Kontraktion (CPS(7)), Schlüpfen (HB) und vollständig geschlüpft (FH).

4. Verfahren nach Anspruch 2, wobei die erste Periode als t4 definiert ist und die zweite Zeitperiode als tEB -t8 definiert ist oder wobei die erste Periode als t5 definiert ist und die zweite Zeitperiode als tEB -t5 definiert ist.

5. Verfahren nach einem der vorhergehenden Ansprüche 2 bis 4, wobei das Verhältnis, das als die zweite Zeitperiode definiert ist, dividiert durch die erste Zeitperiode, ein Indikator für hohe Embryoqualität ist, wenn das Verhältnis größer als ein vorgegebener Wert ist.

6. Verfahren nach einem der vorhergehenden Ansprüche 2 bis 5, wobei eine lange Dauer der ersten Zeitperiode relativ zu einer kurzen Dauer der zweiten Zeitperiode ein Indikator für hohe Embryoqualität ist.

7. Verfahren nach einem der vorhergehenden Ansprüche 5 bis 6, wobei das Blastozystenqualitätskriterium ein Indikator für hohe Embryoqualität ist, wenn das Verhältnis größer oder gleich 1,08 ist.

8. Verfahren nach einem der vorhergehenden Ansprüche 2 bis 7, wobei ein Blastozystenqualitätskriterium die Bestimmung von tM - t8 ist und wobei das Blastozystenqualitätskriterium ein Indikator für hohe Embryoqualität ist, wenn tM - t8 größer oder gleich 27,3 Stunden ist.

9. Verfahren nach einem der vorhergehenden Ansprüche 2 bis 8, wobei ein Blastozystenqualitätskriterium die Bestimmung von tIC ist und wobei das Blastozystenqualitätskriterium ein Indikator für hohe Embryoqualität ist, wenn tIC zwischen 72,4 und 79 Stunden liegt.

10. Verfahren nach einem der vorhergehenden Ansprüche 1 bis 9, wobei ein Blastozystenqualitätskriterium die Bestimmung von tBI - tCPS(2) ist und wobei das Blastozystenqualitätskriterium ein Indikator für hohe Embryoqualität ist, wenn tBI - tCPS(2) größer oder gleich 10,4 Stunden ist.

11. Verfahren nach einem der vorhergehenden Ansprüche 1-10, wobei ein Blastozystenqualitätskriterium die Bestimmung von tCPS(2) - tCPS(1) ist und wobei das Blastozystenqualitätskriterium ein Indikator für hohe Embryoqualität ist, wenn tCPS(2) - tCPS(1) größer oder gleich 7,76 Stunden ist.

12. Verfahren nach einem der vorhergehenden Ansprüche 2 bis 11, wobei ein Blastozystenqualitätskriterium die Bestimmung der absoluten oder relativen 2D-und/oder 3D-Expansion des Blastozysten ist.

13. Verfahren nach einem der vorhergehenden Ansprüche 2 bis 12, wobei der Durchmesser und/oder das Volumen des Embryos zu Beginn der Expansion bestimmt wird und wobei der maximale Durchmesser und/oder das maximale Volumen des Blastozysten vor dem Schlüpfen bestimmt wird und wobei ein Blastozystenqualitätskriterium das Verhältnis der Durchmesser ist und/oder wobei ein Blastozystenqualitätskriterium das Verhältnis der Volumina ist.

14. Verfahren nach Anspruch 1, wobei ein Blastozystenqualitätskriterium eine Bestimmung von Folgendem ist: tCPS(1) und/oder tBI - tCPS(2) und/oder tEB - tCPS(1) und/oder tCPS(2) - tCPS(1), und wobei das Blastozystenqualitätskriterium ein Indikator für hohe Embryoqualität ist, wenn: tCPS(1) kleiner als 107 Stunden ist, tBI - tCPS(2) größer als 8 Stunden ist, tEB - tCPS(1) kleiner als 4 Stunden oder größer als -5 Stunden ist, oder tCPS(2) - tCPS(1) größer als 5 Stunden ist, wobei tCPS(1) die Zeit von der Fertilisierung bis zur ersten Kontraktion ist und tCPS(2) die Zeit von der Fertilisierung bis zur zweiten Kontraktion ist.

## Revendications

1. Procédé de détermination de la qualité d'un embryon humain comprenant la surveillance de l'embryon pendant une période de temps depuis la fertilisation jusqu'au développement du blastocyste au moyen d'un équipement de microscopie accélérée, la détermination des critères de qualité du blastocyste tEB et tBI, où tEB indique un temps depuis la fertilisation jusqu'au développement du blastocyste et tBI indique un temps depuis la fertilisation jusqu'au blastocyste, pour ledit embryon, et sur la base de tEB et tBI, la détermination de la qualité de l'embryon, lesdits critères de qualité du blastocyste constituant un indicateur d'une qualité d'embryon élevée si tEB est inférieur à 122,5 heures et tBI est inférieur à 96,3 heures.

2. Procédé de la revendication 1, comprenant en outre la surveillance du temps depuis la fertilisation jusqu'à un stade de blastocyste, et dans lequel 1) la durée d'une première période de temps depuis la fertilisation jusqu'à ce que la traduction de l'ARNm hérité de la mère dans les blastomères soit achevée et 2) la durée d'une deuxième période de temps depuis l'initiation de la transcription du propre ADN des blastomères jusqu'audit stade de blastocyste sont déterminées, et dans lequel un critère de qualité du blastocyste est le rapport desdites première et deuxième périodes de temps, et sur la base dudit ou desdits critères de qualité du blastocyste la détermination de la qualité de l'embryon.

3. Procédé selon la revendication 2, dans lequel ledit stade de blastocyste est choisi dans le groupe constitué par : la compaction initiale (IC), la morula (M), la différenciation initiale des cellules du trophectoderme (IDT), le blastocyste jeune (ERB), le blastocyste (BI), le développement du blastocyste (EB), la première contraction (CPS(1)), la deuxième contraction (CPS(2)), la troisième contraction (CPS(3)), la quatrième contraction (CPS(4)), la cinquième contraction (CPS(5)), la sixième contraction (CPS(6)), la septième contraction (CPS(7)), l'éclosion (HB), et totalement éclos (FH).

4. Procédé selon la revendication 2, dans lequel ladite première période est définie comme t4 et ladite deuxième période de temps est définie comme tEB - t8 ou dans lequel ladite première période est définie comme t5 et ladite deuxième période de temps est définie comme tEB - t5.

5. Procédé selon l'une quelconque des revendications 2 à 4 précédentes, dans lequel le rapport défini comme la deuxième période de temps divisée par la première période de temps est un indicateur d'une qualité d'embryon élevée si ledit rapport est supérieur à une valeur prédéfinie.

6. Procédé selon l'une quelconque des revendications 2 à 5 précédentes, dans lequel une durée longue de la première période de temps par rapport à une durée courte de la deuxième période de temps est un indicateur d'une qualité d'embryon élevée.

7. Procédé selon l'une quelconque des revendications 5 à 6 précédentes, dans lequel ledit critère de qualité du blastocyste est un indicateur d'une qualité d'embryon élevée si ledit rapport est supérieur ou égal à 1,08.

8. Procédé selon l'une quelconque des revendications 2 à 7 précédentes, dans lequel un critère de qualité du blastocyste est la détermination de tM - t8, et dans lequel ledit critère de qualité du blastocyste est un indicateur d'une qualité d'embryon élevée si tM - t8 est supérieur ou égal à 27,3 heures.

9. Procédé selon l'une quelconque des revendications 2 à 8 précédentes, dans lequel un critère de qualité du blastocyste est la détermination de tIC et dans lequel ledit critère de qualité du blastocyste est un indicateur d'une qualité d'embryon élevée si tIC se situe entre 72,4 et 79 heures.

10. Procédé selon l'une quelconque des revendications 1 à 9 précédentes, dans lequel un critère de qualité du blastocyste est la détermination de tBI - tCPS(2) et dans lequel ledit critère de qualité du blastocyste est un indicateur d'une qualité d'embryon élevée si tBI - tCPS(2) est supérieur ou égal à 10,4 heures.

11. Procédé selon l'une quelconque des revendications 1 à 10 précédentes, dans lequel un critère de qualité du blastocyste est la détermination de tCPS(2) - tCPS(1) et dans lequel ledit critère de qualité du blastocyste est un indicateur d'une qualité d'embryon élevée si tCPS(2) - tCPS(1) est supérieur ou égal à 7,76 heures.

12. Procédé selon l'une quelconque des revendications 2 à 11 précédentes, dans lequel un critère de qualité du blastocyste est la détermination du développement 2D et/ou 3D absolu ou relatif du blastocyste.

13. Procédé selon l'une quelconque des revendications 2 à 12 précédentes, dans lequel le diamètre et/ou le volume de l'embryon au début du développement sont déterminés et dans lequel le diamètre maximal et/ou le volume maximal du blastocyste avant éclosion sont déterminés et dans lequel un critère de qualité du blastocyste est le rapport desdits diamètres et/ou dans lequel un critère de qualité du blastocyste est le rapport desdits volumes.

14. Procédé selon la revendication 1, dans lequel un critère de qualité de blastocyste est une détermination de : tCPS(1), et/ou tBI - tCPS(2), et/ou tEB - tCPS(1), et/ou tCPS(2) - tCPS(1), et dans lequel ledit critère de qualité du blastocyste est un indicateur d'une qualité d'embryon élevée si : tCPS(1) est inférieur à 107 heures, tBI - tCPS(2) est supérieur à 8 heures, tEB - tCPS(1) est inférieur à 4 heures ou supérieur à - 5 heures, ou tCPS(2) - tCPS(1) est supérieur à 5 heures, tCPS(1) étant le temps depuis la fertilisation jusqu'à la première contraction et tCPS(2) étant le temps depuis la fertilisation jusqu'à la deuxième contraction.

Fig. 1

Fig. 2

Fig. 3b

Fig. 3a

Fig. 4b

Fig. 4a

Fig. 5b

Fig. 5a

Fig. 6b

Fig. 6a

Fig. 7b

Fig. 7a

Fig. 8b

Fig. 8a

Fig. 9a

Fig. 9b

Compaction

Not part of compaction

VC(1)                    VC(2)                    VC(3)

Fig. 10a                 Fig. 10b                 Fig. 10c

EP 2 855 664 B1

Fig. 11b

Fig. 11a

Fig. 12

EP 2 855 664 B1

Fig. 13b

Fig. 13a

Fig. 14

Fig. 15

EP 2 855 664 B1

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011025736 A
- DK 201205018 W
- WO 2007144001 A
- DK 2012050236 W
- WO 2007042044 A
- WO 2004056265 A

### Non-patent literature cited in the description

- Human embryonic development after blastomere removal: a time-lapse analysis. **KIRKEGAARD et al.** Human Reproduction. IRL Press, vol. 27, 97-105
- Time-lapse video analysis provides a correlation between early embryo division kinetics and subsequent blastocyst formation and quality. **CRUZ et al.** Human Reproduction; 27th annual meeting of the European Society of Human Reproduction and Embryology. Oxford Journals, 03 July 2011, vol. 26, I167
- **M. MESEGUER et al.** The Use of Morphokinetics as a Predictor of Embryo Implantation. *Human Reproduction,* ISSN 0268-1161, 2658-2671
- Kinetic markers of human embryo quality using time-lapse recordings of IVF/ICSI-fertilized oocytes. **LEMMEN J G et al.** Reproductive Biomedicine Online. Reproductive Healthcare Ltd, vol. 17, 385-391
- **ALIKANI M ; CALDERON G ; TOMKIN G et al.** Cleavage anomalies in early human embryos and survival after prolonged culture in-vitro. *Hum Reprod,* 2000, vol. 15, 2634-2643
- **WONG CC ; LOEWKE KE ; BOSSERT NL et al.** Non-invasive imaging of human embryos before embryonic genome activation predicts development to the blastocyst stage. *Nat Biotechnol,* 2010, vol. 28, 1115-1121
- **KROENER L et al.** The effect of timing of embryonic progression on chromosomal abnormality. *Fertility and Sterility,* 2012, vol. 98 (4), 876-880